# EUROPEAN PATENT SPECIFICATION

(11) **EP 3 784 762 B1**
(45) Date of publication and mention of the grant of the patent: **03.09.2025**
(21) Application number: 19719331.1
(22) Date of filing: 26.04.2019
(51) Int. Cl.: C11B 9/00, C07C 69/02, C07C 43/04

(54) **ESTERS AND ETHERS OF 3-METHYL-PENTANE-DIOL AND UNSATURATED DERIVATIVES THEREOF AND THEIR USE AS AROMA CHEMICAL**
ESTER UND ETHER VON 3-METHYL-PENTAN-DIOL UND UNGESÄTTIGTE DERIVATE DAVON SOWIE DEREN VERWENDUNG ALS AROMACHEMIKALIE
ESTERS ET ÉTHERS DE 3-METHYL-PENTAN-DIOL ET LEURS DÉRIVÉS INSATURÉS ET LEUR UTILISATION EN TANT QU'ARÔME CHIMIQUE

(30) Priority: 27.04.2018 EP 18169757
(43) Date of publication of application: 03.03.2021
(73) Proprietor: BASF SE, 67056 Ludwigshafen am Rhein (DE)
(72) Inventor: BRU ROIG, Miriam, 67056 Ludwigshafen (DE); DANZ, Manuel, 67056 Ludwigshafen (DE); PELZER, Ralf, 68623 Lampertheim (DE); GARLICHS, Florian, 68623 Lampertheim (DE)
(74) Representative: BASF IP Association
(86) International application number: PCT/EP2019/060801
(87) International publication number: WO 2019/207137

(56) References cited:
- EP-A1- 0 950 652
- CH-A5- 600 761
- DE-A1- 1 802 121
- GB-A- 1 424 230
- JP-A- 2016 130 316
- US-A- 3 933 804
- US-A- 3 978 097
- US-A- 4 011 269
- T. A. KAAL, A. YU. ERM, A. U. LINDSAAR, M. R. KAL'YURAND, K. V. LEETS: "INVESTIGATION OF ION-CATALYZED TELOMERIZATION XI. COMPOSITION OF THE PRODUCT FROM TELOMERIZATION OF ISOPRENE WITH CHLOROMETHYL ISOPROPYL ETHER", JOURNAL OF ORGANIC CHEMISTRY OF THE USSR, vol. 11, no. 9, September 1975 (1975-09-01), pages 1819 - 1820, XP009513965
- G. M. KRYAN, A. A. POGOSYAN, E. E. KAPLANYAN, ZH. S. PETROSYAN, G. G. MKRYAN, R. KH. AIRAPETYAN, AND A. A. NAZARYAN: "CHEMISTRY OF ETHERS WITH UNSATURATED RADICALS XXV. CLEAVAGE OF 1,5-DIALKOXY-2-ALKENES. PROTOTROPIC ISOMERIZATION OF 1-ALKOXY-2,4-ALKADIENES AND 2-ALKOXYETHYL 1,3-BUTADIENES", JOURNAL OF ORGANIC CHEMISTRY OF THE USSR, vol. 10, no. 11, November 1974 (1974-11-01), pages 2288 - 2294, XP009513966
- DATABASE pubchem compound [online] 4 December 2011 (2011-12-04), "1,5-Diethoxy-3-methylidenepentane", XP002792353, retrieved from National Center for biotechnology information Database accession no. CID 54440438
- DATABASE PubChem Compound [online] 13 February 2015 (2015-02-13), "3-Methyl-1,5-dipropoxypentane", XP002792354, retrieved from National Center for Biotechnology Information Database accession no. CID 88426276
- DATABASE PubChem Compound [online] 17 March 2015 (2015-03-17), "1,5-Dibutoxy-3-methylpentane", XP002792355, retrieved from National Center for Biotechnology Information Database accession no. CID 91083574
- DATABASE pubchem compound [online] 17 March 2015 (2015-03-17), "(5-Formyloxy-3-methylpentyl) formate", XP002792356, retrieved from national center for biotechnology information Database accession no. CID 91318391
- DATABASE pubchem compound [online] 12 February 2015 (2015-02-12), "(5-Formyloxy-3-methylpentyl) acetate", XP002792357, retrieved from national center for biotechnology information Database accession no. CID 87753826
- DATABASE Pubchem compound [online] 21 May 2013 (2013-05-21), "2-Pentene, 3-methyl-5-(1-methylethoxy)-1-propoxy-, (E)-", XP002792358, retrieved from national center for biotechnology information Database accession no. CID 71333078
- DATABASE pubchem compound [online] 13 February 2015 (2015-02-13), "(5-Methoxy-3-methylpentyl) acetate", XP002792359, retrieved from national center for biotechnology information Database accession no. CID 89397614
- DATABASE pubchem compound [online] 13 February 2015 (2015-02-13), "[(E)-5-Methoxy-3-methylpent-2-enyl] acetate", XP002792360, retrieved from national center for biotechnology information Database accession no. CID 90224847
- DATABASE pubchem compound [online] 7 November 2015 (2015-11-07), "(E)-3-Methyl-5-propan-2-yloxy-1-propoxypent-2-ene", XP002792361, retrieved from national center for biotechnology information Database accession no. CID 92033375
- P. KARRER & J. LEE: "57. Synthese des 6,11-Dimethyl-hexadekan-dions-(2,15)", HELVETICA CHIMICA ACTA, vol. 17, 1934, pages 543 - 549, XP002792362
- DATABASE pubchem compound [online] 15 February 2016 (2016-02-15), "5-Methoxy-3-methylidenepentan-1-ol", XP002792363, retrieved from national center for biotechnology information Database accession no. CID 117268193
- DATABASE Pubchem Compound [online] 26 October 2006 (2006-10-26), "(5-Hydroxy-3-methylpentyl) acetate", XP002792364, retrieved from National center for biotechnology information Database accession no. CID 10997337
- DATABASE Pubchem compound [online] 5 December 2007 (2007-12-05), "(E)-3-Methyl-5-propan-2-yloxypent-2-en-1-ol", XP002792365, retrieved from national center for biotechnology information Database accession no. CID 21515938
- BRYAN R. MOSER: "Preparation and Evaluation of Multifunctional Branched Diesters As Fuel Property Enhancers for Biodiesel and Petroleum Diesel Fuels", ENERGY & FUELS., vol. 28, no. 5, 30 April 2014 (2014-04-30), WASHINGTON, DC, US., pages 3262 - 3270, XP055497783, ISSN: 0887-0624, DOI: 10.1021/ef500482f
- SULTANA SABERA ET AL: "Scandium(III) triflate catalyzed synthesis of primary homoallylic alcohols via carbonyl-ene reaction", TETRAHEDRON LETTERS, ELSEVIER, AMSTERDAM, NL, vol. 54, no. 12, 17 January 2013 (2013-01-17), pages 1576 - 1578, XP028978664, ISSN: 0040-4039, DOI: 10.1016/J.TETLET.2013.01.046
- BRACE N O ED - BARNES JONATHAN C ET AL: "The uncatalysed thermal addition of formaldehyde to olefins", JOURNAL OF THE AMERICAN CHEMICAL SOCIETY, AMERICAN CHEMICAL SOCIETY, US, vol. 77, no. 17, 1 September 1955 (1955-09-01), pages 4666 - 4668, XP002193521, ISSN: 0002-7863

## Description

The present invention relates to the use of esters and/or ethers of 3-methyl-pentane-diol and unsaturated derivates thereof as aroma chemicals, and to aroma chemical compositions comprising at least one ester of 3-methyl-pentane-diol and/or one or more unsaturated derivates thereof and/or at least one ether of 3-methyl-pentane-diol and/or one or more unsaturated derivates thereof. The present invention further relates to composition comprising specific mixtures of such compounds.

### BACKGROUND OF THE INVENTION

Aroma chemicals, especially fragrances, are of great interest especially in the field of cosmetics and cleaning and laundry compositions. Most fragrances of natural origin are expensive, often limited in their available amount and, on account of fluctuations in environmental conditions, are also subject to variations in their content, purity etc. To circumvent these undesirable factors, it is therefore of great interest to create synthetic substances which have organoleptic properties that resemble those of more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should, however, also have additional positive secondary properties, such as e.g. an efficient preparation method, the possibility of providing better sensory profiles as a result of synergistic effects with other fragrances, a higher stability under certain application conditions, a higher extendability, a better staying power, etc.

However, since even small changes in chemical structure bring about massive changes in the sensory properties such as odor and also taste, the targeted search for substances with certain sensory properties such as a certain odor is extremely difficult. The search for new fragrances and flavorings is therefore in most cases difficult and laborious and it is uncertain whether a substance with the desired odor and/or taste will even actually be found.

EP 0908455 describes an odorant compositions which contains macrocycles, such as 15-to 17-memered compounds and processes for manufacturing the same. In particular 3-methyl-1,5-pentandiol diacetate [CAS No. 40065-27-8] is used as reactant for the preaprartion of 3-methyl-5-oxopentanol acetate. However, 3-methyl-1,5-pentandiol diacetate is not mentioned as aroma chemical.

GB 1,424,230 relates to a process for preparing (cyclo)aliphatic/aromatic diethers, which are said to be useful as insecticides. Among many others, 5-isopropoxy-3-methyl-1-pentanol and 5-sec-butoxy-3-methyl-1-pentanol are used as intermediates in the synthesis of the target compounds. No specific property is ascribed to these intermediates.

CH 600761 relates to stilbene derivatives and their use as pesticides. Among many others, 5-isopropoxy-3-methyl-1-pentanol and 1-acetoxy-5-isopropoxy-3-methyl-2-pentene are used as intermediates in the synthesis of the target compounds. No specific property is ascribed to these intermediates.

B. R. Moser describes in Energy&Fuels, 2014, 28, 3262-3270 the preparation of diesters and their use as fuel property enhancers for biodiesel and petroleum diesel fuel. Among others, [3-methyl-5-(3-methylbutanoyloxy)pentyl] 3-methylbutanoate is used. (5-Hydroxy-3-methyl-pentyl) 3-methylbutanoate is used as an intermediate in the preparation thereof.

S. Sultana et al. describe in Tetrahedron Letters 2013, 54, 1576-1578 the Sc(III)-catalyzed synthesis of homoallylic alcohols. Inter alia, 3-methylbut-3-enyl acetate is reacted with paraformaldehyde to give a stereoisomeric mixture of (5-hydroxy-3-methylene-pentyl) acetate, [(E)-5-hydroxy-3-methyl-pent-2-enyl] acetate and [(Z)-5-hydroxy-3-methyl-pent-2-enyl] acetate. No specific property is ascribed to these compounds.

The following compounds are known:
- compound (5-acetoxy-3-methylene-pentyl) acetate (CAS No 40760-37-0),
- compound [(E)-5-acetoxy-3-methyl-pent-3-enyl] acetate (CAS No 1262763-96-1),
- compound [(Z)-5-acetoxy-3-methyl-pent-3-enyl] acetate (CAS No 1262764-00-0),
- compound (5-acetoxy-3-methyl-pentyl) acetate (CAS No. 40065-27-8, EP 0908455),
- compound [(E)-5-formyloxy-3-methyl-pent-3-enyl] formate (CAS No. 53799-56-7),
- compound [(Z)-5-formyloxy-3-methyl-pent-3-enyl] formate (CAS No. 53799-55-6),
- compound (3-methyl-5-propanoyloxy-pentyl) propanoate (CAS No. 1438983-26-6),
- compound [5-(2,2-dimethylpropanoyloxy)-3-methyl-pentyl] 2,2-dimethylpropanoate (CAS No. 762268-77-9),
- compound 1,5-dimethoxy-3-methylene-pentane (CAS No. 79437-43-7),
- compound (E)-1,5-dimethoxy-3-methyl-pent-2-ene (CAS No. 123351-71-3),
- compound (Z)-1,5-dimethoxy-3-methyl-pent-2-ene (CAS No. 123351-70-2),
- compound 1,5-dimethoxy-3-methyl-pentane (CAS No. 4457-73-2),
- compound (Z)-1,5-diethoxy-3-methyl-pent-2-ene (CAS No. 1086266-95-6),
- compound 1,5-diethoxy-3-methyl-pentane (CAS No. 99868-13-0),
- compound (E)-1,5-diisopropoxy-3-methyl-pent-2-ene (CAS No. 116097-01-9),
- compound (Z)-1,5-diisopropoxy-3-methyl-pent-2-ene (CAS No. 116114-80-8),
- compound 1,5-dibutoxy-3-methylene-pentane (CAS No. 79437-42-6),
- compound (E)-1,5-dibutoxy-3-methyl-pent-2-ene (CAS No. 121997-62-4),
- compound (Z)-1,5-dibutoxy-3-methyl-pent-2-en (CAS No. 121997-61-3),
- 5-isopropoxy-3-methyl-1-pentanol,
- 5-sec-butoxy-3-methyl-1-pentanol,
- 1-acetoxy-5-isopropoxy-3-methyl-2-pentene,
- (5-hydroxy-3-methyl-pentyl) 3-methylbutanoate,
- [3-methyl-5-(3-methylbutanoyloxy)pentyl] 3-methylbutanoate,
- (5-hydroxy-3-methylene-pentyl) acetate,
- [(E)-5-hydroxy-3-methyl-pent-2-enyl] acetate,
- [(Z)-5-hydroxy-3-methyl-pent-2-enyl] acetate,
- (5-hydroxy-3-methyl-pentyl) formate.

The following mixtures are known:
- mixture of [(E)-5-acetoxy-3-methyl-pent-3-enyl] acetate and [(Z)-5-acetoxy-3-methyl-pent-3-enyl] acetate (CAS No. 18273-38-6),
- mixture of [(E)-3-methyl-5-propanoyloxy-pent-3-enyl] propanoate and [(Z)-3-methyl-5-propanoyloxy-pent-3-enyl] propanoate (CAS No. 38169-71-0),
- mixture of (E)-1,5-dimethoxy-3-methyl-pent-2-ene and (Z)-1,5-dimethoxy-3-methyl-pent-2-ene (CAS No. 54284-82-1),
- mixture of (Z)-1,5-diethoxy-3-methyl-pent-2-ene and (E)-1,5-diethoxy-3-methyl-pent-2-ene (CAS No. 42173-34-2),
- mixture of (E)-3-methyl-1,5-dipropoxy-pent-2-ene and (Z)-3-methyl-1,5-dipropoxy-pent-2-ene (CAS No. 54284-83-2),
- mixture of (E)-1,5-dibutoxy-3-methyl-pent-2-ene and (Z)-1,5-dibutoxy-3-methyl-pent-2-en (CAS No 79437-47-1),
- mixture of (5-hydroxy-3-methylene-pentyl) acetate, [(E)-5-hydroxy-3-methyl-pent-2-enyl] acetate and [(Z)-5-hydroxy-3-methyl-pent-2-enyl] acetate.

G.M. Kryan et al., J. Org. Chem. of the USSR, 1974, 10(11), 2288-2294 relates inter alia to the cleavage of 1,5-dialkoxy-2-alkenes. Among others, 1,5-dimethoxy-, 1,5-diethoxy- and 1,5-dipropoxy-3-methyl-pent-2-ene are subjected to an elimination reaction to the respective 5-alkoxy-3-methyl-penta-1,3-diene or 2-(2-alkoxyethyl)-but-1,3-diene using KOH as a base. The reaction is carried out neat and is diluted with water after completion.

P. Karrer et al., Helv. Chim. Acta, 1934, 17, 543-549 relates to the synthesis of 6,11-dimethylhexadecane-2,15-dione. In the synthetic route, 5-ethoxy-3-methylpentanol is prepared from the diol, which is reacted first with sodium and then with ethyl iodide, yielding also the diether in small amounts. The reaction is carried out neat and is diluted with ether after completion.

DE 1802121 relates to monoalcohols or monoesters having an alkyl or alkenyl group with at least 10 carbon atoms in the alcohol part, to 1,6-hexanediols carrying alkyl groups in the 3- and 6-positions and the use of these compounds in fragrance compositions.

EP 0950652 A1 relates to monoacetates of a branched, olefinically unsaturated aliphatic or aromatic-aliphatic alcohol and the use thereof in fragrance compositions.

The use of esters and ethers derived from 3-methyl-pentane-diol and unsaturated derivates thereof as aroma chemicals has so far not been described in the prior art.

### SUMMARY OF THE INVENTION

It was an object of the present invention to provide substances exhibiting pleasant organoleptical properties, which can be advantageously used as aroma chemicals. It was a further object of the present invention to provide substances which can be used as an aroma chemical in ready-to-use compositions. In particular, odor-intensive substances having a pleasant odor are sought. Furthermore, these aroma chemicals should be combinable with other aroma chemicals, allowing the creation of novel advantageous sensory profiles. In addition, these aroma chemicals should be obtainable from readily available starting materials, allowing their fast and economic manufacturing, and should not raise toxicological concerns.

It was surprisingly found that these and further objects are achieved by esters and ethers derived from 3-methyl-pentane-diol and unsaturated derivatives thereof.

Accordingly, a first aspect of the present invention relates to the use of a compound of the general formula (I) 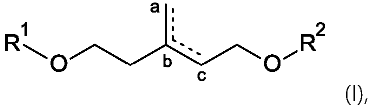 wherein
one of the bonds depicted as -̅ -̅ is a single bond and the other is a double bond or a single bond,
R¹ and R² are identical and are either both C₁-C₄-alkyl, or R¹ is -(C=O)-R³ and R² is -(C=O)-R⁴, where R³ and R⁴ are identical and are hydrogen or C₁-C₄-alkyl,
or of a stereoisomer thereof, or of a mixture of stereoisomers thereof, or of a mixture of different compounds (I), as an aroma chemical.

In particular, the present invention relates to the use of a compound of the general formula (I.a) or (I.b) 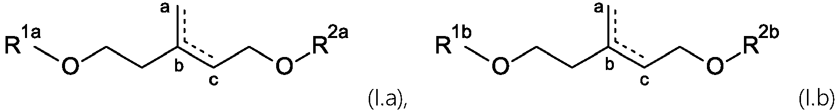 wherein
one of the bonds depicted as -̅ -̅ is a single bond and the other is a double bond or a single bond,
R^{1a} and R^{2a} are identical and are both C₁-C₄-alkyl,
R^{1b} is -(C=O)-R³, and
R^{2b} is -(C=O)-R⁴,
where R³ and R⁴ are identical and are hydrogen or C₁-C₄-alkyl,
or of a stereoisomer thereof, or of a mixture of stereoisomers thereof, or of a mixture of different compounds (I.a) or a mixture of different compounds (I.b), as an aroma chemical.

The present invention further relates to an aroma chemical compositions comprising at least one compound of formula (I), (I.a) or (I.b), a stereoisomer thereof or a mixture of stereoisomers thereof, as defined above, and at least one further component selected from the group consisting of aroma chemicals different from compounds (I), (I.a) and (I.b) and non-aroma chemical carriers,
where the carrier materials are liquid or oil-like, or solid (including wax-like) carrier materials;
where the liquid or oil-like carrier materials are selected from methanol, ethanol, ethylene glycol, glycerol, diglycerol, propylene glycol, dipropylene glycol, 1,2-butylene glycol, hexamethylcyclotrisiloxane, decamethylcyclopentasiloxane, diethylene glycol monoethyl ether, diethyl phthalate, isopropyl myristate, triethyl citrate, benzyl benzoate, fractionated coconut oil, tetradecyl acetate, tetradecyl lactate, esters of glycerol with melting temperatures below 20°C, and isopropyl myristate.

It was further found that the compounds of the general formula (I), (I.a) or (I.b) generally exhibit a pleasant and characteristic odor and can be used to produce fragranced compositions, in particular fragranced ready-to-use compositions. In addition, they can advantageously be combined with other aroma chemicals different from compounds (I), (I.a) and (I.b) to create new scent profiles.

Therefore, described, but not according to the invention, is a method of preparing a fragranced composition, in particular a fragranced ready-to-use composition, comprising incorporating at least one compound of formula (I), (I.a) or (I.b), a stereoisomer thereof, a mixture of stereoisomers thereof, into a composition, in particular into a ready-to-use composition, which is to be fragranced.

The invention also relates to the use of a compound of formula (I) (I.a) or (I.b), a stereoisomer thereof, a mixture of stereoisomers thereof or a mixture of different compounds of formula (I), (I.a) or (I.b), as defined above, for modifying the scent character of a fragranced composition, in particular of a fragranced ready-to-use composition.

The invention relates moreover to a composition comprising a mixture of at least two compounds selected from the group consisting of isomers of formulae (I.1), (I.2) and (I.3), 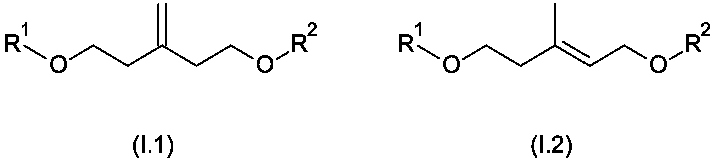 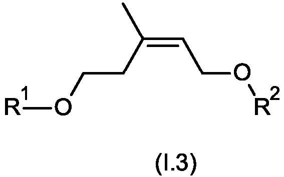
where in all compounds of formulae (I.1), (I.2) and (I.3) present in the mixture all radicals R¹ and R² are identical and are either C₁-C₄-alkyl, or all radicals R¹ are -(C=O)-R³, all radicals R² are -(C=O)-R⁴, and all radicals R³ and R⁴ are identical and are hydrogen or C₁-C₄-alkyl, preferably hydrogen, methyl or ethyl;
comprising each of the compounds present in the mixture in an amount of from 1 - 99 % by weight, with the proviso that the weight of the compounds of formulae (I.1), (I.2) and (I.3) present in said mixture adds up to 100%;
except for following mixtures:
   - mixture of [(E)-5-acetoxy-3-methyl-pent-3-enyl] acetate and [(Z)-5-acetoxy-3-methyl-pent-3-enyl] acetate,
   - mixture of [(E)-3-methyl-5-propanoyloxy-pent-3-enyl] propanoate and [(Z)-3-methyl-5-propanoyloxy-pent-3-enyl] propanoate,
   - mixture of (E)-1,5-dimethoxy-3-methyl-pent-2-ene and (Z)-1,5-dimethoxy-3-methyl-pent-2-ene,
   - mixture of (E)-1,5-diethoxy-3-methyl-pent-2-ene and (Z)-1,5-diethoxy-3-methyl-pent-2-ene,
   - mixture of (E)-3-methyl-1,5-dipropoxy-pent-2-ene and (Z)-3-methyl-1,5-dipropoxy-pent-2-ene, and
   - mixture of (E)-1,5-dibutoxy-3-methyl-pent-2-ene and (Z)-1,5-dibutoxy-3-methyl-pent-2-ene.

The compounds of formula (I), (I.a) or (I.b), their stereoisomers, the mixtures of their stereoisomers and mixtures of different compounds of formula (I), (I.a) or (I.b) possess advantageous organoleptic properties, in particular a pleasant odor. Therefore, they can be favorably used as an aroma chemical for example in perfume compositions, body care compositions (including cosmetic compositions), products for oral and dental hygiene, hygiene articles, cleaning compositions (including dishwashing compositions), textile detergent compositions, compositions for scent dispensers, foods, food supplements, pharmaceutical compositions, crop protection compositions and other ready-to-use compositions.

By virtue of their physical properties, the compounds of formula (I), (I.a) or (I.b), their stereoisomers, the mixtures of their stereoisomers and mixtures of different compounds of formula (I), (I.a) or (I.b) have particularly good, virtually universal solvent properties for other fragrances and other customary ingredients in fragranced ready-to-use compositions such as, in particular, perfume compositions. Therefore, the compounds of formula (I), (I.a) or (I.b), their stereoisomers, the mixtures of their stereoisomers and mixtures of different compounds of formula (I), (I.a) or (I.b) are favorably combinable with other aroma chemicals, allowing, in particular, the creation of perfume compositions having novel advantageous sensory profiles.

Furthermore, the compounds of formula (I), (I.a) or (I.b), their stereoisomers or the mixtures of stereoisomers thereof can be produced in good yields and purities by a one-step or a two-step synthesis, starting from readily available starting compounds, such as inexpensive 3-methyl-pentane-diol and/or unsaturated derivates thereof. Thus, the compounds of formula (I), (I.a) or (I.b), their stereoisomers or the mixtures of stereoisomers thereof can be produced on large scale and in a simple and cost-efficient manner. The same applies of course to the production of mixtures of different compounds of formula (I), (I.a) or (I.b).

### DETAILED DESCRIPTION OF THE INVENTION

Depending on the meaning of the bonds symbolized as -̅ -̅, the compound (I) can be depicted as having one of the following formulae (I.1) to (I.4):

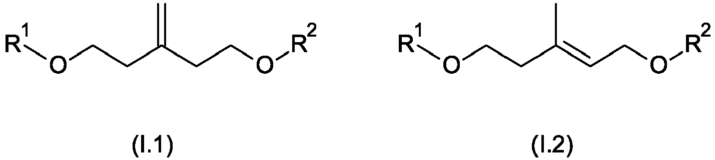

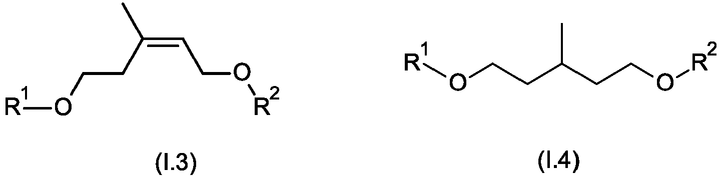

In compound (I.1), the bond -̅ -̅ between carbon atoms a and b is a double bond, and the bond -̅ -̅ between carbon atoms b and c is a single bond. In compound (I.2), the bond -̅ -̅ between carbon atoms a and b is a single bond, the bond -̅ -̅ between carbon atoms b and c is a double bond and the arrangement of the radicals -CH₂CH₂OR¹ and -CH₂OR² at carbon atoms b and c is such that compound (I) is present as an E isomer (further details to stereoisomers see below). In compound (I.3), the bond - - between carbon atoms a and b is a single bond, the bond -̅ -̅ between carbon atoms b and c is a double bond and the arrangement of the radicals -CH₂CH₂OR¹ and -CH₂OR² at carbon atoms b and c is such that compound (I) is present as a Z isomer. In compound (I.4), both bonds -̅ -̅ between carbon atoms a and b and between carbon atoms b and c are single bonds.

In the following, the compounds of the formula (I.1) are also termed as "exo" compounds (exo), the compounds of the formula (I.2) are also termed as E compounds (E), the compounds of the formula (I.3) are also termed as Z compounds (Z), and the compounds of the formula (I.4) are also termed as saturated compounds (sat).

In the context of the present invention, the expression "C₁-C₄-alkyl" refers to methyl, ethyl, n-propyl, isopropyl, n-butyl, sec-butyl, isobutyl and tert.-butyl. Preferably, the expression "C₁-C₄-alkyl" refers to C₁-C₃-alkyl, i.e. to methyl, ethyl, n-propyl and isopropyl, and in particular to C₁-C₂-alkyl, i.e. to methyl and ethyl.

The term "aroma chemical" denotes a substance which is used to obtain a sensory impression, to be more precise an olfactory or flavor impression, in particular a fragrance impression. The term "olfactory" denotes an odor impression without any positive or negative judgement, while the term "fragrance" (also termed "perfume" or "scent") is connected to an odor impression which is generally felt as pleasant. A flavor induces a taste impression.

The term "aroma chemical composition", as used herein, refers to a composition which induces an olfactory or flavor impression, and which in particular induces an odor impression, specifically a fragrance impression.

Likewise, the term "fragranced ready-to-use composition", as used herein, refers to a ready-to-use composition which induces an odor, in particular a fragranced impression.

The term "odor-intensive substances" refers to substances or aroma chemicals exhibiting intense odor impressions. Intense odor impressions are to be understood as meaning those properties of aroma chemicals which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

"Pleasant odor", "pleasant odor impression", "pleasant odiferous properties", "odor impression felt as pleasant" and similar terms are hedonistic expressions which describe the niceness and conciseness of an odor impression conveyed by an aroma chemical. The more general hedonistic expressions "advantageous sensory properties" or "advantageous organoleptic properties" describe the niceness and conciseness of an organoleptic impression conveyed by an aroma chemical. In terms of the present invention, the terms "organoleptic" and "sensory" relate to olfactory or flavor properties. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "red berries": the odor would then be concisely of "red berries". If this red berries odor were very pleasant because the odor is reminiscent, for example, of sweet, fully ripe red berries, the odor would be termed "nice" and/or "sweet". However, the odor of a typically tart red berries can also be concise. If both reactions arise upon smelling the substance, in the example thus a nice and concise red berries odor, then this substance has particularly advantageous sensory properties.

Preferably, the compound of formula (I) or a stereoisomer thereof or a mixture of stereoisomers thereof or a mixture of different compounds of formula (I) as defined above and below is used for imparting an olfactory impression. In particular, the compound of formula (I) or a mixture thereof or a stereoisomer thereof or a mixture of stereoisomers thereof as defined above is used as a fragrance.

Apart from individual compounds (I), be it in form of achiral compounds (I) (e.g. if both bonds depicted as -̅ -̅ are single bonds and R¹ and R² are identical and achiral) or in form of pure stereoisomers (see definition below), and apart from various stereoisomer mixtures, mixtures of different compounds (I) can be used. The compounds in such mixtures differ for example in being saturated (i.e. both bonds depicted as -̅ -̅ are single bonds) or unsaturated (i.e. one of the bonds depicted as -̅ -̅ is a double bond), in being positional isomers with respect to the position of the double bond, i.e. in having the double bond between carbon atoms a and b or between carbon atoms b and c, and/or in having different radicals R¹ and/or R². Such mixtures can of course also contain the components in form of various stereoisomers.

The term "stereoisomers" denotes isomers which have the the same molecular formula and sequence of bonded atoms, but differ in the three-dimensional orientations of their atoms in space. It encompasses both optical isomers, such as enantiomers or diastereomers, the latter existing due to more than one stereogenic center in the molecule, as well as geometrical isomers (cis/trans or Z/E isomers) as a specific form of diastereomers.

In the first place, in terms of the present invention, the term "stereoisomers" refers to the E and Z isomers of the unsaturated compounds of formula (I), (I.a) or (I.b), i.e. of those compounds in which the bond between carbon atoms b and c depicted as -̅ -̅ is a double bond. The E/Z configuration describes the absolute stereochemistry of double bonds in organic chemistry. Each substituent on a double bond is assigned a priority. If the two groups of higher priority are on opposite sides of the double bond, the bond is assigned the configuration E. If the two groups of higher priority are on the same side of the double bond, the bond is assigned the configuration Z.

For the purpose of illustration only, the E and Z isomers of [5-formyloxy-3-methyl-pent-3-enyl] formate are shown below:

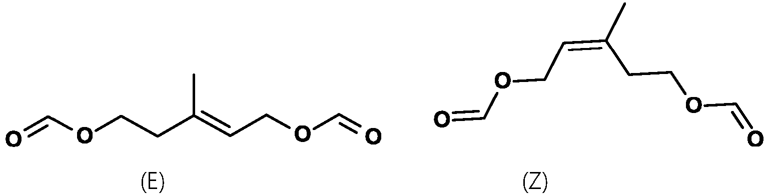

A general illustration of E and Z stereoisomers of compounds (I) is given in the formulae (I.2) (E isomer) and (I.3) (Z isomer) shown above and below. To be stereoisomers, it is of course a prerequisite that R¹ has the same meaning in (I.2) and (I.3) and R² has the same meaning in (I.2) and (I.3).

For better legibility, in the present application, compounds (I), (I.a) or (I.b) which may be present in form of Z and E stereoisomers are generally shown as E stereoisomers only. This does however not restrict the compounds to the E stereoisomers (unless explicitly expressed otherwise, e.g. by expressly terming the compounds as (I.2) or (I.3) or as E or Z), but includes the Z isomers and mixtures of the E und Z isomers.

Secondly, the term "stereoisomer" refers also to optical isomers of the saturated compounds of formula (I), (I.a) or (I.b). Optical isomers are enantiomers or diastereomers which exist due to more than one stereogenic center in the molecule. It refers to all optical isomers in pure form, e.g. to all enantiomers or diastereomers in pure form. An enantiomer is one of two stereoisomers that are non-superposable mirror images of each other. If in compounds (I), (I.a) or (I.b) both bonds depicted as -̅ -̅ are single bonds and R¹ and R² have different meanings (which is not according to the invention), carbon atom b carrying the methyl group is a stereogenic center. If R¹ and R² do not contain a stereogenic center, such compounds (I), (I.a) or (I.b) can be present in form of the pure 3R or the pure 3S enantiomer.

For the purpose of illustration only, the 3R- and 3S-isomers of (5-hydroxy-3-methylpentyl) formate are shown below:

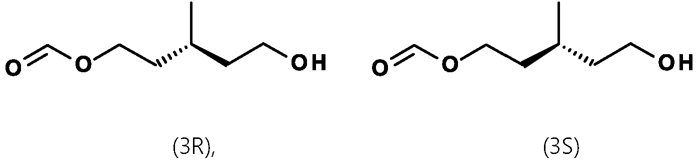

Thirdly, stereogenic centers may also be present in radicals R¹, R², R³ or R⁴, e.g. if these are sec-butyl.

Mixtures of stereoisomers are for example racemic and non-racemic mixtures of the enantiomers or diastereomers of the compounds (I), (I.a) or (I.b). In this context, the term "diastereomers" encompasses both diastereomers existing due to more than one stereogenic center in the molecule and geometric isomers, i.e. E and Z isomers.

The term "isomers" denotes compounds with identical elemental formulas but distinct structures. The term encompasses both structural isomers and stereoisomers. Stereoisomers have been defined above.

Structural isomers of compounds (I) are in particular positional isomers with respect to the position of the double bond. To be more precise, compounds (I) in which the double bond is between carbon atoms a and b are a structural isomer of compounds (I) in which the double bond is between carbon atoms b and c. Thus, compounds of the formula (I.1) are positional isomers of compounds (I.2) or (I.3). To be isomers, it is of course a prerequisite that R¹ has the same meaning in (I.1) and (I.2) or (I.3), and R² has the same meaning in (I.1) and (I.2) or (I.3).

Depending on the presence and arrangement of the double bond, the compounds of the formula (I) can exist as individual "exo"-isomers (I.1) (exo), E-isomers (I.2) (E) or Z-isomers (I.3) (Z), or as saturated compounds (I.4) (sat): 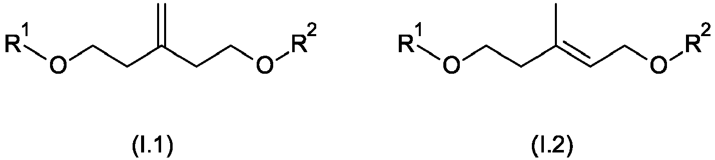 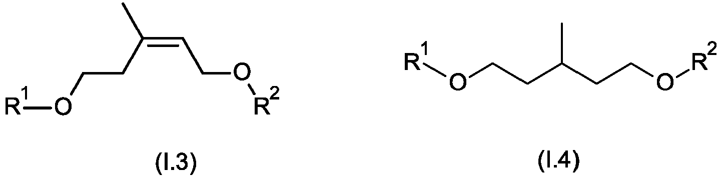 or as various mixtures thereof, such as exo/E/Z/sat mixtures or exo/E/Z isomer mixtures.

The present invention thus relates to the use of the exo isomers, i.e. (I.1), the use of the E-isomers, i.e. (I.2), the use of the Z-isomers, i.e. (I.3), the use of the saturated compounds, i.e. (O.4), and also the use of various mixtures thereof, such as E/Z isomer mixtures, i.e. (I.2, I.3), exo/E isomer mixtures, i.e. (I.1, I.2), exo/Z isomer mixtures, i.e. (I.1, I.3), exo/E/Z isomer mixtures, i.e. (I.1, I.2, I.3), or also exo/Z/E/sat mixtures thereof, i.e. (I.1, I.2, I.3, I.4). Thus, if not stated otherwise, the expressions "compounds I", "compounds of the general formula I" and the like, as used herein, refers to the pure exo isomers, the pure E isomers, the pure Z isomer, the pure saturated compound as well as to various mixtures thereof, such as the above-listed mixtures, in which the isomers/compounds are present in equal quantities or contain one of the isomers/compounds in excess or one or more isomers/compounds are absent.

As a matter of course, in an isomer mixture R' in all isomers has the same meaning and R² in all isomers has the same meaning. Preferably, in mixtures containing the saturated compound (I.4) and one or more of (I.1), (I.2) and (I.3), too, R¹ in all isomers/compounds has the same meaning and R² in all isomers/compounds has the same meaning.

The pure exo-isomers, the pure E-isomers, the pure Z-isomers and the pure saturated compounds as well as the various (isomer) mixtures of the compounds (I) have all advantageous organoleptic properties. Thus, the pure exo isomers, the pure E-isomers, the pure Z-isomers and the pure saturated compounds as well as the various (isomer) mixtures of the compounds (I) are equally suitable for use as aroma chemicals.

Also suitable for use as aroma chemicals are mixtures which contain compounds (I) which differ from each other in the radicals R¹ and/or R². Such mixtures can be formed in the production process, for instance if the alkylation or acylation of the starting diol is not complete (if the target compound is a diether or diester). Further details will become evident in context with the description of the preparation process of compounds (I). Mixtures of compounds (I) which differ from each other in the radicals R¹ and/or R² and which arise from incomplete or undesired reaction preferably contain the compounds obtained from incomplete conversion (e.g. monoethers or monoesters in which one of R¹ or R² is H, if the production of diethers or diesters is intended) in only minor amounts, such as at most 25% by weight, preferably at most 10% by weight, more preferably at most 5% by weight, in particular at most 2% by weight, specifcally at most 1% by weight, based on the total weight of all compounds (I) in the mixture.

On the other hand, usually the basic scent profile of the ether compounds of the general formula (I) differs from the scent profile of the ester compounds of the general formula (I).

Therefore a first embodiment of the present invention relates to the use of an ether compound of the general formula (I), where in formula (I)
- R¹: is C₁-C₄-alkyl and
- R²: is C₁-C₄-alkyl,
where R¹ and R² are identical.

The compounds (I) of this first embodiment encompass the the diethers of 3-methyl-pentane-diol and unsaturated derivates thereof.

Preferred for use are ether compounds of the general formula (I), wherein R¹ and R² identical and are C₁-C₃-alkyl.

More preferred for use are ether compounds of the general formula (I), wherein R¹ and R² are identical and selected from the group consisting of methyl, ethyl, n-propyl, isopropyl and n-butyl.

Even more preferred for use are ether compounds of the general formula (I), wherein R¹ and R² are identical and are methyl, ethyl or n-butyl.

Particularly preferred for use are ether compounds of the general formula (I), wherein R¹ and R² are ethyl.

Especially preferred for use are
- 1,5-diethoxy-3-methyl-pentane,
- 1,5-diethoxy-3-methylene-pentane,
- (E)-1,5-diethoxy-3-methyl-pent-2-ene,
- (Z)-1,5-diethoxy-3-methyl-pent-2-ene,
- 1,5-dimethoxy-3-methyl-pentane,
- 1,5-dimethoxy-3-methylene-pentane,
- (E)-1,5-dimethoxy-3-methyl-pent-2-ene,
- (Z)-1,5-dimethoxy-3-methyl-pent-2-ene,
- 1,5-dibutoxy-3-methyl-pentane,
- 1,5-dibutoxy-3-methylene-pentane,
- (E)-1,5-dibutoxy-3-methyl-pent-2-ene,
- (Z)-1,5-dibutoxy-3-methyl-pent-2-ene and
- mixtures thereof.

In another embodiment a composition comprising a mixture of at least two compounds selected of a compound of formulae (I.1), (I.2), (I.3) and (I.4) is used 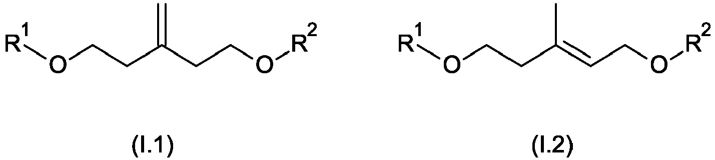 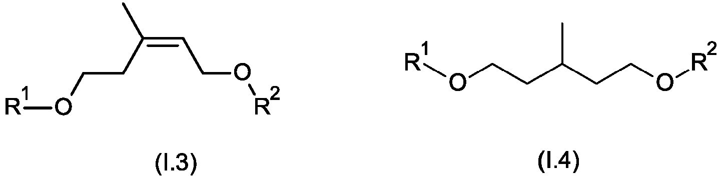
wherein R¹ and R² have one of the general or preferred meanings as defined above in context with the first embodiment,
comprising each of the compounds present in the mixture in an amount of 1 - 99 % by weight, with the proviso that weight of the compounds of formulae (I.1), (I.2), (I.3) and (I.4) in said mixture adds up to 100%.

Preferred for use are compositions comprising a mixture of at least two compounds selected from the compounds of formulae (I.1), (I.2) and (I.3), comprising each of the compounds present in the mixture in an amount of 1 - 99 % by weight, with the proviso that the weight of the compounds of formulae (I.1), (I.2) and (I.3) in said mixture adds up to 100%.

More preferred for use are compositions comprising a mixture of all three compounds of formulae (I.1), (I.2) and (I.3), comprising each of the compounds in an amount of 1 - 99 % by weight, with the proviso that the weight of the compounds of formulae (I.1), (I.2) and(I.3) in said mixture adds up to 100%, wherein the molar ratio of (I.1):(I.2):(I.3) is in the range of from 90:5:5 to 10:45:45, preferably from 80:10:10 to 20:40:40, especially from 55:22.5:22.5 to 45:27.5:27.5, and is more especially approximately 50:25:25. The term "approximately" takes account of measurement errors which may occur in determining the ratio and includes a deviation of ±10%, preferably of ±5%.

A second embodiment of the present invention relates to the use of an ester compound of the general formula (I), where in formula (I)
- R¹: is -(C=O)-R³,
- R²: is -(C=O)-R⁴, and
- R³ and R⁴: are as defined above (i.e. are identical and are hydrogen or C₁-C₄-alkyl).

The compounds (I) of this second embodiment encompass the diesters of 3-methyl-pentane-diol and unsaturated derivates thereof.

Preferred for use are ester compounds of the general formula (I), wherein R³ and R⁴ are identical and selected from the group consisting of hydrogen and C₁-C₃-alkyl.

More preferred for use are ester compounds of the general formula (I), wherein R³ and R⁴ are identical and selected from the group consisting of hydrogen, methyl and ethyl.

Especially preferred for use are
- (3-formyloxy-2-methyl-propyl) formate,
- 2-(formyloxymethyl)allyl formate,
- [(E)-3-formyloxy-2-methyl-allyl] formate,
- [(Z)-3-formyloxy-2-methyl-allyl] formate,
- (3-acetoxy-2-methyl-propyl) acetate,
- 2-(acetoxymethyl)allyl acetate,
- [(E)-3-acetoxy-2-methyl-allyl] acetate,
- [(Z)-3-acetoxy-2-methyl-allyl] acetate,
- (2-methyl-3-propanoyloxy-propyl) propanoate,
- 2-(propanoyloxymethyl)allyl propanoate,
- [(E)-2-methyl-3-propanoyloxy-allyl] propanoate,
- [(Z)-2-methyl-3-propanoyloxy-allyl] propanoate, and
- mixtures thereof.

In another embodiment the invention relates to the use of a composition comprising a mixture of at least two compounds selected of a compound of formulae (I.1), (I.2), (I.3) and (I.4) 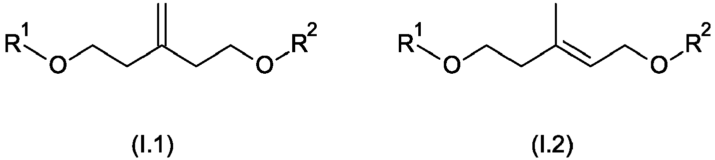 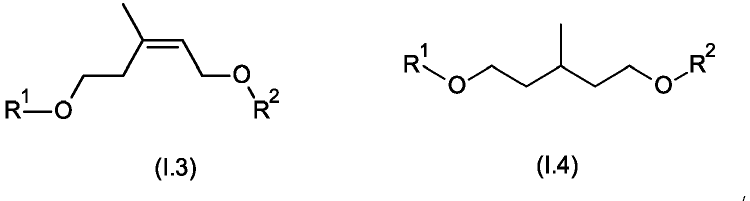 wherein R¹ and R² have one of the general and preferred meanings as defined above in context with the second embodiment, comprising each of the compounds present in the mixture in an amount of 1 - 99 % by weight, with the proviso that the weight of the compounds of formulae (I.1), (I.2), (I.3) and (I.4) in said mixture adds up to 100%.

Preferred for use are compositions comprising a mixture of at least two compounds selected from the compounds of formulae (I.1), (I.2) and (I.3), comprising each of the compounds present in the mixture in an amount of 1 - 99 % by weight, with the proviso that the weight of the compounds of formulae (I.1), (I.2) and (I.3) in said mixture adds up to 100%.

More preferred for use are compositions comprising a mixture of all three compounds of formulae (I.1), (I.2) and (I.3), comprising each of the compounds in an amount of 1 - 99 % by weight, with the proviso that the weight of the compounds of formulae (I.1), (I.2) and(I.3) in said mixture adds up to 100%, wherein preferably the molar ratio of (I.1):(I.2):(I.3) is in the range of from 90:5:5 to 10:45:45, more preferably from 80:10:10 to 20:40:40, especially from 55:22.5:22.5 to 45:27.5:27.5, and is more especially aproximately 50:25:25. The term "approximately" takes account of measurement errors which may occur in determining the ratio and includes a deviation of ±10%, preferably of ±5%.

In a particular embodiment of the present invention, the ether and ester compounds of the general formula (I) are essentially present in the form of their exo isomers (I.1).

In another particular embodiment of the present invention, the ether and ester compounds of the general formula (I) are essentially present in the form of their E-isomers (I.2).

In another particular embodiment of the present invention, the ether and ester compounds of the general formula (I) are essentially present in the form of their Z-isomers (I.3).

In another particular embodiment of the present invention, the ether and ester compounds of the general formula (I) are essentially present in the form of its sat compounds (I.4), i.e. a saturated derivative in which both bonds depicted as -̅ -̅ are single bonds.

In this connection, the term "essentially" means that the exo, E, Z or the sat derivative of the particular compound is present in an amount of at least 90% by weight, preferably in an amount of at least 95% by weight, in particular in an amount of at least 98% by weight, based on the total amount (weight) of the exo, E, Z and the sat derivatives.

Typically, the compounds (I) are produced as exo/E/Z or as exo/E/Z/sat mixtures. The separation of the exo, E, Z and the sat derivatives can be difficult and laborious or even cannot be achieved completely with reasonable effort.

Thus, a preferred embodiment of the present invention relates to the use of a compound of the general formula (I), as defined above and below, where the compound of the general formula (I) is present in the form of an exo/E/Z isomer mixture which may also contain the saturated derivative sat (being thus an exo/E/Z/sat mixture). In particular, use is made of an exo/E/Z isomer mixture.

In one embodiment, in these mixtures, the four derivatives can be present in equal amounts or almost equal amounts, e.g. in a exo/E/Z/sat molar ratio of 25:25:25:25 or 31:23:23:23 or 23:31:23:23 or 23:23:31:23 or 23:23:23:31, or one of the derivatives, i.e. either the exo, E, Z or sat derivative, can be present in excess, e.g. one of the derivatives can be present in exo/E/Z/sat molar ratio of 91:3:3:3, 3:91:3:3, 3:3:91:3 or:3:3:3:91. In a particular embodiment, the saturated derivative sat, if at all present, is present in minor amounts only. In a specific embodiment the molar ratio of exo-isomer : E-isomer: Z-isomer : sat-derivative is from 48:25:25:2 to 49:25:25:1.

In another embodiment in these mixtures, the three exo/E/Z isomers can be present in equimolar amounts of 33.3:33.3:33.3, or one of the isomers, i.e. either the exo, E or Z isomer, can be present in excess, e.g. one of the isomers can be present in a exo/E/Z or E/exo/Z or E/Z/exo or Z/exo/E or Z/E/exo molar isomer ratio of 90:9:1, 90:5:5, 80:10:10, 60:20:20, 50:25:25, 40:30:30, where the isomer ratio is preferably from 75:12.5:12.5 to 45:27.5:27.5, and is specifically approximately 50:25:25. The term "approximately" takes account of measurement errors which may occur in determining the ratio and includes a deviation of ±10%, preferably of ±5%.

A preferred embodiment of the present invention relates to the use of a composition comprising a mixture of at least two compounds (I) selected from the compounds of formulae (I.1), (I.2) and (I.3), where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of >0% by weight, based on the total weight of (I.1), (I.2) and (I.3), and that the weight of compound (I.1), (I.2) and (I.3) present in the mixture adds up to 100 %.

A more preferred embodiment of the present invention relates to the use of a composition comprising a mixture of all three compounds of formulae (I.1), (I.2) and (I.3), where
- (I.1) is present in an amount of from 25 to 75 weight %, preferably from 30 to 60 weight %, especially from 35 to 55 weight %, based on the total weight of (I.1), (I.2) and (1.3),
- (I.2) is present in an amount of from 15 to 35 weight %, preferably from 18 to 30 weight %, especially from 20 to 28 weight %, based on the total weight of (I.1), (I.2) and (1.3),
- (I.3) is present in an amount of from 15 to 35 weight %, preferably from 18 to 30 weight %, especially from 20 to 28 weight %, based on the total weight of (I.1), (I.2) and (1.3),
with the proviso that the weight of compound (I.1), (I.2) and (I.3) adds up to 100 %.

A particular embodiment is the use of a composition comprising a mixture of all three compounds of the general formulae (I.1), (I.2), and (I.3) comprising each of the compounds in an amount of 1 - 99 % by weight, with the proviso that the weight of the compounds of formulae (I.1), (I.2), and (I.3) adds up to 100%, wherein the ratio of (I.1):(I.2):(I.3) is in the range of from 90:5:5 to 10:45:45, preferably from 80:10:10 to 20:40:40, especially from 55:22.5:22.5 to 45:27.5:27.5, and more especially is approximately 50:25:25. Again, the term "approximately" takes account of measurement errors which may occur in determining the ratio and includes a deviation of ±10%, preferably of ±5%.

A preferred embodiment of the present invention relates to the use of a composition comprising a mixture of at least two ether compounds selected from the compounds of formulae (I.1), (I.2) and (I.3) wherein R¹ and R² are as defined in the above first embodiment relating to ether compounds, where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of > 0% by weight, based on the total weight of (I.1), (I.2) and (I.3), and that the weight of compound (I.1), (I.2) and (I.3) present in the mixture adds up to 100 %.

A more preferred embodiment of the present invention relates to the use of a composition comprising a mixture of all three ether compounds selected from the compounds of formulae (I.1), (I.2) and (I.3) wherein R¹ and R² are as defined in the above first embodiment relating to ether compounds, where
- (I.1) is present in an amount of from 25 to 75 weight %, preferably from 30 to 60 weight %, especially from 35 to 55 weight %, based on the total weight of (I.1), (I.2) and (1.3),
- (I.2) is present in an amount of from 15 to 35 weight %, preferably from 18 to 30 weight %, especially from 20 to 28 weight %, based on the total weight of (I.1), (I.2) and (1.3),
- (I.3) is present in an amount of from 15 to 35 weight %, preferably from 18 to 30 weight %, especially from 20 to 28 weight %, based on the total weight of (I.1), (I.2) and (1.3),
with the proviso that the weight of compound (I.1), (I.2) and (I.3) adds up to 100 %.

A special embodiment is the use of a composition comprising a mixture of all three ether compounds of the general formulae (I.1), (I.2), and (I.3) wherein R¹ and R² are as defined in the above first embodiment comprising each of the ether compounds in an amount of 1 - 99 % by weight, with the proviso that the weight of the ether compounds of formulae (I.1), (I.2), and (I.3) adds up to 100%, wherein the ratio of (I.1):(I.2):(I.3) is in the range of from 90:5:5 to 10:45:45, preferably from 80:10:10 to 20:40:40, especially from 55:22.5:22.5 to 45:27.5:27.5, and more especially is approximately 50:25:25. Again, the term "approximately" takes account of measurement errors which may occur in determining the ratio and includes a deviation of ±10%, preferably of ±5%.

A preferred embodiment of the present invention relates to the use of a composition comprising a mixture of at least two ester compounds selected from the compounds of formulae (I.1), (I.2) and (I.3) wherein R¹ and R² are as defined in the above second embodiment relating to ester compounds, where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of > 0% by weight, based on the total weight of (I.1), (I.2) and (I.3), and that the weight of compound (I.1), (I.2) and (I.3) present in the mixture adds up to 100 %.

A more preferred embodiment of the present invention relates to the use of a composition comprising a mixture of all three ester compounds selected from the compounds of formulae (I.1), (I.2) and (I.3) wherein R¹ and R² are as defined in the above second embodiment relating to ester compounds, where
- (I.1) is present in an amount of from 25 to 75 weight %, preferably from 30 to 60 weight %, especially from 35 to 55 weight %, based on the total weight of (I.1), (I.2) and (1.3),
- (I.2) is present in an amount of from 15 to 35 weight %, preferably from 18 to 30 weight %, especially from 20 to 28 weight %, based on the total weight of (I.1), (I.2) and (1.3),
- (I.3) is present in an amount of from 15 to 35 weight %, preferably from 18 to 30 weight %, especially from 20 to 28 weight %, based on the total weight of (I.1), (I.2) and (1.3),
with the proviso that the weight of compound (I.1), (I.2) and (I.3) adds up to 100 %.

A special embodiment is the use of a composition comprising a mixture of all three ester compounds of the general formulae (I.1), (I.2), and (I.3) wherein R¹ and R² are as defined in the above second embodiment comprising each of the compounds in said in an amount of 1 - 99 % by weight, with the proviso that the weight of the compounds of formulae (I.1), (I.2), and (I.3) adds up to 100%, wherein the ratio of (I.1):(I.2):(I.3) is in the range of from 90:5:5 to 10:45:45, preferably from 80:10:10 to 20:40:40, especially from 55:22.5:22.5 to 45:27.5:27.5, and more especially is approximately 50:25:25. Again, the term "approximately" takes account of measurement errors which may occur in determining the ratio and includes a deviation of ±10%, preferably of ±5%.

Furthermore, the compounds (I), (I.a) or (I.b) can be used as aroma chemicals over a wide range of purity, as long as the impurities do not have a significant detrimental effect on the scent of the compounds (I), (I.a) or (I.b). For the use as aroma chemicals, according to the invention, the purity of the compounds (I), (I.a) or (I.b) is therefore not specifically limited. Preferably, the compounds (I), (I.a) or (I.b) have a purity of at least 70%, in particular of at least 90% and especially of at least 95%.

Due to the method of their preparation starting from 3-methyl-pentane-diol and/or unsaturated derivates thereof, the diether compounds of the general formula (I) or (I.a) may comprise minor amounts of monoether compounds (I-OH), and the diester compounds of the general formula (I) or (I.b) may comprise minor amounts of monoester compounds (II-OH) 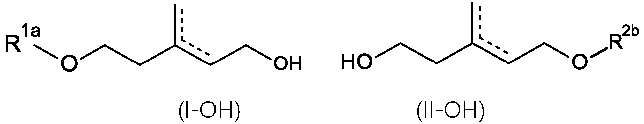 wherein R^{1a} and R^{2b} have one of the meanings given above.

Preferably, the amounts of mono-substituted compounds (I-OH) or (II-OH), which can be comprised in the diether and the diester compounds of the general formula (I) are less than 25% by weight, more preferably less than 10% by weight, even more preferably less than 5% by weight, in particular less than 1% by weight, based on the total amount of the compound (I).

In a particularly preferred embodiment of the present invention the diether and diester compounds of the general formula (I) do not comprise mono-substituted compounds (I-OH) or (II-OH), respectively.

The aforesaid preferred embodiments can be combined with one another as desired.

Accordingly, in a particular preferred embodiment, the present invention relates to the use of a compound of the general formula (I), which has a purity of at least 95%, and/or is present in the form of a exo/E/Z isomer mixture as mentioned above.

Specifically, the present invention relates to the use of a compound of the general formula (I), which has a purity of at least 95%, and/or is present in the form of a exo/E/Z isomer mixture with a weight ratio as mentioned above, preferably with a weight ratio of exo:E:Z of 55:22.5:22.5 to 45:27.5:27.5, and more specifically with a weight ratio of exo:E:Z of approximately 50:25:25, where in the compound (I) R¹ and R² are identical, and where in all isomers of the exo/E/Z mixture R¹ and R² are identical. The term "approximately" takes account of measurement errors which may occur in determining the ratio and includes a deviation of ±10%, preferably of ±5%.

As already mentioned, the compound of formula (I) or a stereoisomer thereof or a mixture of stereoisomers thereof or a mixture of different compounds of formula (I) as defined above and below is preferably used for imparting an olfactory impression. In particular, the compound of formula (I) or a mixture thereof or a stereoisomer thereof or a mixture of stereoisomers thereof as defined above is used as a fragrance.

In particular, an isomer mixture of compounds of formula (I.1), (I.2) and (I.3) (see above definition), where in each case R¹ and R² are -C(=O)CH₃, is used to impart a sweet, powdery, terpenic, fruity note; or is used to produce a scent with a sweet, powdery, terpenic, fruity note. Specifically, in this mixture, the compounds (I.1), (I.2) and (I.3), where in each case R¹ and R² are -C(=O)CH₃, are present in a weight ratio of ca. 50:25:25.

In particular, a compound (I.1), wherein R¹ and R² are -C(=O)CH₃, is used to impart a fruity, sweet, rubber, musty, dusty note; or is used to produce a scent with a fruity, sweet, rubber, musty, dusty note.

In particular, a compound (I.2), wherein R¹ and R² are -C(=O)CH₃, is used to impart a fruity, woody, sweet note; or is used to produce a scent with a fruity, woody, sweet note.

In particular, a compound (I.4) (see above definition), wherein R¹ and R² are -C(=O)CH₃, is used to impart a sweet, fruity note; or is used to produce a scent with a sweet, fruity note.

In particular, an isomer mixture of compounds of formula (I.1), (I.2) and (I.3), where in each case R' and R² are -C(=O)CH₂CH₃, is used to impart a sweet, red berries note; or is used to produce a scent with a sweet, red berries note. Specifically, in this mixture, the compounds (I.1), (I.2) and (I.3), where in each case R¹ and R² are -C(=O)CH₂CH₃, are present in a weight ratio of ca. 50:25:25.

In particular, an isomer mixture of compounds of formula (I.1), (I.2) and (I.3), where in each case R¹ and R² are -CH₃, is used to impart a herbal, algae, green, watery, fruity note; or is used to produce a scent with a herbal, algae, green, watery, fruity note. Specifically, in this mixture, the compounds (I.1), (I.2) and (I.3), where in each case R¹ and R² are -CH₃, are present in a weight ratio of ca. 50:25:25.

In particular, an isomer mixture of compounds of formula (I.1), (I.2) and (I.3), where in each case R¹ and R² are -CH₂CH₃, is used to impart a green, herbal, raw potato, green banana note; or is used to produce a scent with a green, herbal, raw potato, green banana note. Specifically, in this mixture, the compounds (I.1), (I.2) and (I.3), where in each case R¹ and R² are -CH₂CH₃, are present in a weight ratio of ca. 50:25:25.

In particular, an isomer mixture of compounds of formula (I.1), (I.2) and (I.3), where in each case R¹ and R² are -CH₂CH₂CH₂CH₃, is used to impart a cedar, citrus, warm, herbal note; or is used to produce a scent with a cedar, citrus, warm, herbal note. Specifically, in this mixture, the compounds (I.1), (I.2) and (I.3), where in each case R¹ and R² are - CH₂CH₂CH₂CH₃, are present in a weight ratio of ca. 50:25:25.

In particular, a compound (O.4), wherein R¹ and R² are -CH₂CH₃, is used to impart a green, banana, apple (ripe) note; or is used to produce a scent with a green, banana, apple (ripe) note.

The above "ca." terms take account of measurement errors which may occur in determining the ratio and includes a deviation of ±10%, preferably of ±5%.

The monoether compounds of the general formula (I) (not according to the invention) can efficiently be prepared by alkylating the compound (II) or mixtures thereof 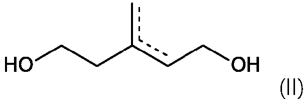 using an alkylation reagent R¹-X, wherein R¹ has one of the meanings given above and X represents a leaving group, selected from halogen, such as Cl, Br, I, and sulfonates, such as tosylate, mesylate, triflate or nonaflate, typically in the presence of a base.

Suitable bases are typically selected from inorganic bases and organic bases.

Suitable inorganic bases that can be used in this alkylation reaction are for example alkali metal carbonates, e.g. Li₂CO₃, Na₂CO₃, K₂CO₃ or Cs₂CO₃, alkali metal hydroxides, e.g. LiOH, NaOH or KOH, and hydride donors, e.g. NaH, LiAlH₄ or NaBH₄.

Suitable organic bases that can be used in this alkylation reaction are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The alkylation reaction is performed under conventional alkylation reaction conditions that are well known to the skilled person.

Typically, compound (II) or mixtures thereof are reacted with sub-equimolar or almost equimolar amounts, e.g. 0.7, 0.8, 0.9, or 0.95 equivalents, of the alkylation reagent R¹-X to give the mono-substituted compound (I-OH), which is further subjected to a purification step in order to remove unwanted by-products or impurities, such as residual starting material or the corresponding di-substituted compounds.

Generally, the purification step is performed by using common purification methods, such as crystallization, distillation or chromatographic methods, e.g. column chromatography or high performance liquid chromatography.

The preparation of diether compounds of the general formula (I), where the radicals R¹ and R² are identical, is typically performed by reacting of a compound (II) or mixtures thereof with at least two equivalents, e.g. 2.0, 2.1, 2.5 or 3.0 equivalents, of the alkylation reagent R¹-X or R²-X, respectively, wherein the radicals R¹, R² and X have one of the meanings given above, and the obtained raw product is subsequently subjected to a purification step, as defined above. In case where only minor amounts of impurities and by-products are present in the raw-product mixture the purification step can also be performed via a simple extractive workup.

Generally, the ester compounds of the general formula (I) can efficiently be prepared by reacting a compound (II) or mixtures thereof with the carboxylic acid R³-COOH or with the carboxylic acid R⁴-COOH, wherein R³ and R⁴ have one of the meanings given above, or an acid anhydride thereof, or an acid halide of the formulae R³-(C=O)X' or R⁴-(C=O)X', where X' is a halogen, such as Cl, Br or I, typically Cl or Br. The reaction is typically performed in the presence of an esterification catalyst or a base.

Suitable esterification catalysts that can be applied in this reaction are well known to the skilled person. Suitable esterification catalysts are for example metal based catalysts, e.g. iron, cadmium, cobalt, lead, zinc, antimony, magnesium, titanium and tin catalysts in the form of metals, metal oxides or metal salts, such as metal alcoxylates; mineral acids, such as sulfuric acid, hydrochloric acid or phosphoric acid; or organic sulfonic acids, such as methane sulfonic acid or para-toluene sulfonic acid.

Suitable bases are for example organic bases, as defined above.

Alternatively, the ester compounds of the general formula (I) can be prepared by reacting a compound (II) or mixtures thereof with an acid halide of the formula R³-(C=O)X' or R⁴-(C=O)X', wherein R³ and R⁴ have one of the meanings given above and X' is halogen, such as Cl, Br or I, typically Cl or Br, in the presence of an organic base.

Suitable organic bases are as defined above.

The individual reaction conditions for the preparation of the ester compounds of the general formula (I) are well known to the skilled person.

For the preparation of diester compounds of the general formula (I), where the radicals R³ and R⁴ are identical, compound (II) or a mixtures thereof is typically reacted with at least two equivalents, e.g. 2.0, 2.1, 2.5 or 3.0 equivalents, of the carboxylic acid R³-COOH or R⁴-COOH, respectively, an anhydride thereof or a mixture of said carboxylic acid with an anhydride thereof. Alternatively, compound (II) or mixtures thereof are typically reacted with at least two equivalents of the acid halide R³-(C=O)X' or R⁴-(C=O)X', respectively, in the presence of an organic base. The obtained raw product is subsequently subjected to a purification step, as defined above. In case where only minor amounts of impurities and by-products are present in the raw-product mixture the purification step can also be performed via a simple extractive workup.

The starting material, compound (II) or mixtures thereof, is a diol building block that is frequently used as a monomer for the synthesis of polymeric materials. Thus, compound (II) or mixtures thereof are readily available from commercial sources. Alternatively, compound (II) or mixtures thereof can also by synthesized in large quantities using processes that are well described in the art, e.g. via the dimerization of dimethylketene.

### Compositions:

The below remarks apply both to the aroma chemical composition according to the present invention as well as to the use according to the present invention.

The compounds of formula (I), mixtures thereof, the stereoisomers thereof or a mixture of stereoisomers thereof can be used in a wide range of compositions, such as ready-to-use compositions. The olfactory properties, the substance properties (such as solubility in customary solvents and compatibility with further customary constituents of such compositions), as well as the toxicological acceptability of the compounds of formula (I), underline their particular suitability for the stated use purposes and compositions.

Furthermore, the compounds of the general formula (I) exhibit advantageous secondary properties.

For example, the compounds of the general formula (I) can provide better sensory profiles as a result of synergistic effects with other fragrances, which means that they can provide a booster effect for other fragrances. They are therefore suitable as boosters for other fragrances.

Booster effect means that the substances enhance and intensify, in perfumery formulations, the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione^{®} (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compounds of the general formula (I), the stereoisomers thereof or a mixture of stereoisomers thereof are generally used in an amount of 0.1 - 20% by weight, preferably in an amount of 0.5 to 5% by weight, in particular in an amount of from 0.6 to 3% by weight, based on the total weight of the fragrance mixture.

Furthermore, the compounds of the general formula (I), the stereoisomers thereof or a mixture of stereoisomers thereof can have further positive effects on the composition itself, where they are applied in. For example, the compounds of the general formula (I), the stereoisomers thereof or a mixture of stereoisomers thereof can enhance the overall performance of the composition, such as the stability, e.g. the formulation stability, the extendability or the staying power of the composition.

Accordingly, a further embodiment of the invention therefore relates to the use of a compound of the general formula (I), a stereoisomer thereof, a mixture of stereoisomers thereof or a mixture of different compounds (I), as an aroma chemical in a ready-to-use composition.

The term "ready-to-use composition", as used herein, refers to a composition which is intended to be applied or used on its own by the final user.

Generally, the ready-to-use composition, in which the aroma chemicals of the present invention, i.e. the compounds of the general formula (I), as defined above, can be applied, are fragranced ready-to-use compositions.

Fragranced ready-to-use composition, in which the aroma chemicals of the present invention, i.e. the compounds of the general formula (I), as defined above, can be applied, are for example compositions used in personal care, compositions used in home care, compositions used in industrial applications as well as compositions used in other applications, such as pharmaceutical compositions or crop protection compositions.

Preferably, the ready-to-use compositions are selected from perfume compositions, body care compositions (including cosmetic compositions), products for oral and dental hygiene, hygiene articles, cleaning composition (including dishwashing compositions), textile detergent compositions, compositions for scent dispensers, foods, food supplements, pharmaceutical compositions and crop protection compositions.

The compounds (I), the stereoisomers thereof, the stereoisomer mixtures thereof or the double bond isomers thereof are used as an aroma chemical, preferably as a fragrance, in the above compositions.

In particular, an isomer mixture of compounds of formula (I.1), (I.2) and (I.3) (see definition below), where in each case R¹ and R² are -C(=O)CH₃, is used to impart a sweet, powdery, terpenic, fruity note to the above-listed compositions. Specifically, in the mixture, the compounds (I.1), (I.2) and (I.3), where in each case R¹ and R² are -C(=O)CH₃, are present in a weight ratio of ca. 50:25:25.

In particular, a compound (I.1), wherein R¹ and R² are -C(=O)CH₃, is used to impart a fruity, sweet, rubber, musty, dusty note to the above-listed compositions.

In particular, a compound (I.2), wherein R¹ and R² are -C(=O)CH₃, is used to impart a fruity, woody, sweet note to the above-listed compositions.

In particular, a compound (I.4) (see below definition), wherein R¹ and R² are -C(=O)CH₃, is used to impart a sweet, fruity note to the above-listed compositions.

In particular, an isomer mixture of compounds of formula (I.1), (I.2) and (I.3) (see definition below), where in each case R¹ and R² are -C(=O)CH₂CH₃, is used to impart a sweet, red berries note to the above-listed compositions. Specifically, in the mixture, the compounds (I.1), (I.2) and (I.3), where in each case R¹ and R² are -C(=O)CH₂CH₃, are present in a weight ratio of ca. 50:25:25.

In particular, an isomer mixture of compounds of formula (I.1), (I.2) and (I.3) (see definition below), where in each case R¹ and R² are -CH₃, is used to impart a herbal, algae, green, watery, fruity note to the above-listed compositions. Specifically, in the mixture, the compounds (I.1), (I.2) and (I.3), where in each case R¹ and R² are -CH₃, are present in a weight ratio of ca. 50:25:25.

In particular, an isomer mixture of compounds of formula (I.1), (I.2) and (I.3) (see definition below), where in each case R¹ and R² are -CH₂CH₃, is used to impart a green, herbal, raw potato, green banana note to the above-listed compositions. Specifically, in the mixture, the compounds (I.1), (I.2) and (I.3), where in each case R¹ and R² are -CH₂CH₃, are present in a weight ratio of ca. 50:25:25.

In particular, an isomer mixture of compounds of formula (I.1), (I.2) and (I.3) (see definition below), where in each case R¹ and R² are -CH₂CH₂CH₂CH₃, is used to impart a cedar, citrus, warm, herbal note to the above-listed compositions. Specifically, in the mixture, the compounds (I.1), (I.2) and (I.3), where in each case R¹ and R² are -CH₂CH₂CH₂CH₃, are present in a weight ratio of ca. 50:25:25.

In particular, a compound (I.4) (see below definition), wherein R¹ and R² are -CH₂CH₃, is used to impart a green, banana, apple (ripe) note to the above-listed compositions.

The above "ca." terms take account of measurement errors which may occur in determining the ratio and includes a deviation of ±10%, preferably of ±5%.

Perfume compositions can be selected from fine fragrances, air fresheners in liquid form, gel-like form or a form applied to a solid carrier, aerosol sprays, scented cleaners, perfumed candles and oils, such as lamp oils or oils for massage.

Exemples for fine fragrances are perfume extracts, Eau de Parfums, Eau de Toilettes, Eau de Colognes, Eau de Solide and Extrait Parfum and the like.

Body care compositions include cosmetic compositions, and can be selected from aftershaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, aftershave creams and lotions and tanning creams and lotions, powders, hydrogels, hair care products, such as e.g. hairsprays, hair gels, setting hair lotions, hair conditioners, hair shampoo and permanent and semi-permanent hair colorants, hair shaping compositions, such as cold waves and hair smoothing compositions, hair tonics, hair creams and hair lotions, deodorants and antiperspirants, such as e.g. underarm sprays, roll-ons, deodorant sticks and deodorant creams, products of decorative cosmetics, such as e.g. eye-liners eye-shadows, nail varnishes, make-ups, lipsticks and mascara.

Products for oral and dental hygiene can be selected from toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, bath and sanitary cleaners, scouring milk, solid and liquid toilet cleaners, powder and foam carpet cleaners, dishwashing detergents for hand and machine dishwashing, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers and facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract.

Food supplements may be found in many forms such as tablets, capsules, softgels, gelcaps, liquids, or powders.

Pharmaceutical compositions comprise compositions, which are intended for use in the diagnosis, cure, mitigation, treatment, or prevention of disease as well as articles (other than food) intended to affect the structure or any function of the body of man or other animals.

Crop protection compositions comprise compositions, which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, antiinflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, polyols, surfactants, electrolytes, organic solvents or silicone derivatives.

As mentioned above, it was further found that the compounds of the general formula (I) generally exhibit a pleasant and characteristic odor and can be used to produce fragranced ready-to-use compositions and/or they can advantageously be combined with other aroma chemicals different from compounds (I) to create new scent profiles.

Accordingly, a specific embodiment of the present invention relates to the use of a compound of formula (I), a stereoisomer thereof or a mixture of stereoisomers thereof for modifying the scent character of a fragranced ready-to-use composition.

The present invention further relates to aroma chemical compositions comprising at least one compound of formula (I), a stereoisomer thereof or a mixture of stereoisomers thereof, as defined above, and at least one further component selected from the group consisting of further aroma chemicals different from compounds (I) and non-aroma chemical carriers;
where the carrier materials are liquid or oil-like or solid (including wax-like) carrier materials;
where the liquid or oil-like carrier materials are selected from methanol, ethanol, ethylene glycol, glycerol, diglycerol, propylene glycol, dipropylene glycol, 1,2-butylene glycol, hexamethylcyclotrisiloxane, decamethylcyclopentasiloxane, diethylene glycol monoethyl ether, diethyl phthalate, isopropyl myristate, triethyl citrate, benzyl benzoate, fractionated coconut oil, tetradecyl acetate, tetradecyl lactate, esters of glycerol with melting temperatures below 20°C, and isopropyl myristate.

This includes aroma chemical compositions comprising at least one compound of formula (I), a stereoisomer thereof or a mixture of stereoisomers thereof, as defined above, and at least one further aroma chemical different from compounds (I).

Further included are aroma chemical compositions comprising at least one compound of formula (I), a stereoisomer thereof or a mixture of stereoisomers thereof, as defined above, and at least one non-aroma chemical carrier.

Also included are aroma chemical compositions comprising at least one compound of formula (I), a stereoisomer thereof or a mixture of stereoisomers thereof, as defined above, at least one further aroma chemical different from compounds (I) and at least one non-aroma chemical carrier.

Preferably, the aroma chemical compositions comprise at least one compound of formula (I), a stereoisomer thereof or a mixture of stereoisomers thereof, as defined above, at least one further aroma chemical different from compounds (I) and at least one non-aroma chemical carrier.

The further aroma chemical different from compounds (I) can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat'), dihydromyrcenol (2,6-dimethyl-7-octen-2-ol), methyl dihydrojasmonate (preferably with a content of cis isomer of more than 60% by weight) (Hedione^{®}, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lysmeral^{2a}), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat'), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone'), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide'), 15-cyclopentadecanolide (Macrolide'), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol (Florol⁹), 2-ethyl-4-(2,2,3-trimethyl-3-cyclopenten-1-yl)-2-buten-1-ol (Sandolen'), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone'), 2,6-dimethyl-5-hepten-1-al (Melonal²), borneol, 3-(3-isopropylphenyl)butanal (Florhydral²), 2-methyl-3-(3,4-methylenedioxyphenyl)-propanal (Helional³), 3-(4-ethylphenyl)-2,2-dimethylpropanal (Florazon'), 7-methyl-2H-1,5-benzodioxepin-3(4H)-one (Calone), 3,3,5-trimethylcyclohexyl acetate (preferably with a content of cis isomers of 70% by weight) or more and 2,5,5-trimethyl-1,2,3,4,4a,5,6,7-octahydronaphthalen-2-ol (Ambrinol S¹).

Where trade names are given above, these refer to the following sources:
¹ trade name of Symrise GmbH, Germany;
^{2a} trade name of BASF SE, Germany;
² trade name of Givaudan AG, Switzerland;
³ trade name of International Flavors & Fragrances Inc., USA;
⁵ trade name of Danisco Seillans S.A., France;
⁹ trade name of Firmenich S.A., Switzerland;
¹⁰ trade name of PFW Aroma Chemicals B.V., the Netherlands.

Further aroma chemicals with which the compounds of formula (I), the stereoisomers thereof or a mixture of stereoisomers thereof, as defined above, can be combined e.g. to give a composition according to the invention can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene;
camphene; caryophyllene; cedrene; farnesene; limonene; longifolene; myrcene; ocimene; valencene; (E,Z)-1,3,5-undecatriene; styrene; diphenylmethane;
aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
taliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
cyclic and macrocyclic ketones such as e.g. 4-tert-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclohexadecen-1-one; 7-cyclohexadecen-1-one; (7/8)-cyclohexadecen-1-one; 9-cycloheptadecen-1-one; cyclopentadecanone; cyclohexadecanone;
cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
esters of cycloaliphatic alcohols such as e.g. 1-cyclohexylethyl crotonate;
esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl-2-methyl-1,3-dioxolane-2-acetate;
araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
aromatic and araliphatic aldehydes such as e.g. benzaldehyde; phenylacetaldehyde; 3-phenylpropanal; hydratropaaldehyde; 4-methylbenzaldehyde; 4-methylphenyl-acetaldehyde; 3-(4-ethylphenyl)-2,2-dimethylpropanal; 2-methyl-3-(4-isopropylphenyl)-propanal; 2-methyl-3-(4-tert-butylphenyl)propanal; 2-methyl-3-(4-isobutylphenyl)-propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
aromatic and araliphatic ketones such as e.g. acetophenone; 4-methylacetophenone; 4-methoxyacetophenone; 4-tert-butyl-2,6-dimethylacetophenone; 4-phenyl-2-butanone; 4-(4-hydroxyphenyl)-2-butanone; 1-(2-naphthalenyl)ethanone; 2-benzofuranyl-ethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropyl-pyrazine; 2-isobutyl-3-methoxypyrazine;
phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

Typically, the at least one non-aroma chemical carrier is a compound, a mixture of compounds or other additives, which have no or no noteworthy sensory properties. The non-aroma chemical carrier serves for the dilution and/or the fixing of the aroma chemical(s), i.e. the compounds of formula (I) and optionally one or more further aroma chemical different from compounds (I), as defined above, comprised in the aroma chemical composition.

Suitable carrier materials can be liquid or oil-like carrier materials as well as wax-like or solid carrier materials.

Suitable liquid or oil-like carrier materials are selected, for example, from water, alcohols, such as methanol or ethanol, aliphatic diols and polyols with a melting temperature below 20°C, such as ethylene glycol, glycerol, diglycerol, propylene glycol or dipropylene glycol and 1,2-butylene glycol, cyclic siloxanes, such as hexamethylcyclotrisiloxane or decamethylcyclopentasiloxane, diethylene glycol monoethyl ether, diethyl phthalate, isopropyl myristate, triethyl citrate, benzyl benzoate, vegetable oils, such as fractionated coconut oil or esters of fatty alcohols with melting temperatures below 20°C, such as tetradecyl acetate or tetradecyl lactate, esters of glycerol with melting temperatures below 20°C, and alkyl esters of fatty acids with melting temperatures below 20°C, such as isopropyl myristate.

In the composition according to the invention comprising at least one compound of formula (I), a stereoisomer thereof or a mixture of stereoisomers thereof, and at least one further component selected from the group consisting of aroma chemicals different from compounds (I) and non-aroma chemical carriers, where the carrier materials are liquid or oil-like, or solid (including wax-like) carrier materials, the liquid or oil-like carrier materials are selected from methanol, ethanol, ethylene glycol, glycerol, diglycerol, propylene glycol, dipropylene glycol, 1,2-butylene glycol, hexamethylcyclotrisiloxane, decamethylcyclopentasiloxane, diethylene glycol monoethyl ether, diethyl phthalate, isopropyl myristate, triethyl citrate, benzyl benzoate, fractionated coconut oil, tetradecyl acetate, tetradecyl lactate, esters of glycerol with melting temperatures below 20°C, and isopropyl myristate.

Suitable wax-like or solid carrier materials are selected, for example, from fatty alcohols with melting temperatures below 20°C, such as myristyl alcohol, stearyl alcohol or cetyl alcohol, polyols with melting temperatures above 20°C, fatty acid esters with fatty alcohols which have a melting temperature of above 20°C, such as lanolin, beeswax, carnauba wax, candelilla wax or Japan wax, waxes produced from petroleum, such as hard paraffin, water-insoluble porous minerals, such as silica gel, silicates, for example talc, microporous crystalline aluminosilicates (zeolites), clay minerals, for example bentonite, or phosphates, for example sodium tripolyphosphate, paper, cardboard, wood, textile composite or nonwoven materials made of natural and/or synthetic fibers.

Suitable carrier materials are also selected, for example, from water-soluble polymers, such as polyacrylic acid esters or quaternized polyvinylpyrrolidones, or water-alcohol-soluble polymers, such as specific thermoplastic polyesters and polyamides. The polymeric carrier material can be present in various forms, e.g. in the form of a gel, a paste, solid particles, such as microcapsules, or brittle coatings.

Preferably, the aroma chemical composition is selected from fragranced ready-to-use compositions, as defined above.

Generally, the total amount of the at least one compound of formula (I), a stereoisomer thereof or a mixture of stereoisomers thereof in the aroma chemical compositions according to the present invention are typically adapted to the particular intended use or the intended application and can, thus, vary over a wide range. As a rule, the customary standard commercial use amounts for scents are used.

Accordingly, the total amount of the at least one compound of formula (I), a stereoisomer thereof or a mixture of stereoisomers thereof in the composition is in the range of from 0.001 to 99.9% by weight, preferably in the range of from 0.01 to 90% by weight, more preferably in the range of from 0.05 to 80 % by weight, even more preferably in the range of from 0.1 to 60 % by weight, in particular in the range of from 0.1 to 40 % by weight, based on the total weight of the composition.

In one embodiment of the invention, the total amount of the at least one compound of formula (I), a stereoisomer thereof or a mixture of stereoisomers thereof in the composition is in the range of from 0.001 to 5 weight%, preferably from 0.01 to 2 weight%, based on the total weight of the composition.

A further embodiment of the invention is directed to a composition comprising at least one compound of formula (I), a stereoisomer thereof or a mixture of stereoisomers thereof and at least one component selected from the group consisting of surfactants, emollients and solvents.

One embodiment of the invention is directed to a composition comprising at least one compound of formula (I), a stereoisomer thereof or a mixture of stereoisomers thereof and at least one solvent.

In the context of the present invention, a "solvent" serves for the dilution of the compound of formula (I), a stereoisomer thereof or a mixture of stereoisomers thereof, as defined above. Some solvents have fixing properties at the same time.

The one or more solvent(s) can be present in the composition from 0.01 to 99% by weight based on the composition. In a preferred embodiment of the invention, the composition comprise 0.1 to 90 % by weight, preferably 0.5 to 80 % by weight of solvent(s) based on the composition. The amount of solvent(s) can be chosen depending on the composition. In one embodiment of the invention, the composition comprises 0.05 to 10 % by weight, preferably 0.1 to 5 % by weight, more preferably 0.2 to 3 % by weight based on the composition. In one embodiment of the invention, the composition comprises 20 to 70 % by weight, preferably 25 to 50 % by weight of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compounds of formula (I), the stereoisomers thereof or a mixture of stereoisomers thereof are suitable for use in surfactant-containing compositions. According to their characteristic scent profiles, the compounds of formula (I), the stereoisomers thereof or a mixture of stereoisomers thereof can especially be used for the perfuming of surfactant-containing compositions such as, for example, cleaners (in particular laundry cleaners and all-purpose cleaners).

One embodiment of the invention is therefore directed to a composition comprising at least one compound of formula (I), a stereoisomer thereof or a mixture of stereoisomers thereof and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C₈-C₁₈ alkyl or acyl group, contain at least one free amino group and at least one -COOH- or -SO₃H-group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C₁₂-C₁₈ alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart^{®} series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising at least one compound of formula (I), a stereoisomer thereof or a mixture of stereoisomers thereof and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15% by weight based on the composition.

The oil components may be selected, for example, from Guerbet alcohols based on fatty alcohols con taining 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl ole- ate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol@ CC), Guerbet carbonates based on fatty alcohols containing 6 to 18 and preferably 8 to 10 carbon atoms, esters of benzoic acid with linear and/or branched C₆ to C₂₂-alcohols (for example Finsolv^{®} TN), linear or branched, symmetrical or nonsymmetrical dialkyl ethers containing 6 to 22 carbon atoms per alkyl group such as, for example, dicaprylyl ether (Cetiol^{®} OE), ring opening products of epoxidized fatty acid esters with polyols and hydrocarbons or mixtures thereof.

### Method of preparation:

Fragranced ready-to-use composition can be prepared by a method comprising incorporating at least one compound of formula (I), a stereoisomer thereof or a mixture of stereoisomers thereof, as defined above, into a ready-to-use composition.

This method comprises the incorporation of at least one compound of formula (I), a stereoisomer thereof or a mixture of stereoisomers thereof, to a ready-to-use composition, which has no or no notable sensory properties, on order to provide a specific odor and/or a specific flavor to this ready-to-use composition. In addition, this method also comprises the modification of the odor and/or the flavor of a ready-to-use composition, which already has notable sensory properties, by incorporating at least one compound of formula (I), a stereoisomer thereof or a mixture of stereoisomers thereof, into said ready-to-use composition.

Preferred ready-to-use compositions are those mentioned above.

The total amount of the at least one compound of formula (I), a stereoisomer thereof or a mixture of stereoisomers thereof, that is incorporated in the ready-to-use compositions highly depends on the intended use or the intended application and can, thus, vary over a wide range. Typical amounts of the at least one compound of formula (I), a stereoisomer thereof or a mixture of stereoisomers thereof that is incorporated in the ready-to-use compositions are those as defined above for the compositions.

The compounds of formula (I), the stereoisomers thereof or a mixture of stereoisomers thereof used according to the invention, the compositions obtainable by the above method of the invention, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the compounds of formula (I), the stereoisomers thereof or a mixture of stereoisomers thereof, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compounds of formula (I), the stereoisomers thereof or a mixture of stereoisomers thereof with a suitable wax-like substance and by extrusion with subsequent solidification, optionally in a suitable solvent, e.g. isopropanol.

The aroma chemical composition, in particular a fragranced composition, especially a fragranced ready-to-use composition, can be prepared by a method comprising incorporating at least one compound of formula (I), a stereoisomer thereof or a mixture of stereoisomers thereof into an aroma chemical composition, in particular into a fragranced composition, especially into a fragranced ready-to-use composition.

For example, the method can be carried out by mixing at least one compound of formula (I), a stereoisomer thereof or a mixture of stereoisomers thereof, as defined above, and at least one further compound selected from the group consisting of aroma chemicals different from compounds (I) and non-aroma chemical carriers.

Modifying the scent character of an aroma chemical composition, in particular of a fragranced composition, especially of a fragranced ready-to-use composition, can be carried out by a method comprising incorporating at least one compound of formula (I), a stereoisomer thereof or a mixture of stereoisomers thereof into an aroma chemical composition, in particular into a fragranced composition, especially into a fragranced ready-to-use composition.

Preparing a perfume composition, body care composition, product for oral and dental hygiene, hygiene article, cleaning composition, textile detergent composition, composition for scent dispensers, food, food supplement, pharmaceutical composition or crop protection composition can be carried out by a method comprising including the one or more compounds of formula (I), the stereoisomers thereof or the mixture of stereoisomers thereof as defined above or in a perfume composition, body care composition, product for oral and dental hygiene, product for oral and dental hygiene, hygiene article, cleaning composition, textile detergent composition, composition for scent dispensers, food, food supplement, pharmaceutical composition or crop protection composition.

Imparting a sweet, powdery, terpenic, fruity note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including an isomer mixture of compounds of formula (I.1), (I.2) and (I.3), where in each case R¹ and R² are -C(=O)CH₃, in a perfume composition, body care composition, product for oral and dental hygiene, hygiene article, cleaning composition, textile detergent composition, composition for scent dispensers, food, food supplement, pharmaceutical composition or crop protection composition. Specifically, in the mixture, the compounds (I.1), (I.2) and (I.3), where in each case R¹ and R² are -C(=O)CH₃, are present in a weight ratio of ca. 50:25:25.

Imparting a fruity, sweet, rubber, musty, dusty note to a perfume composition, body care composition, product for oral and dental hygiene, hygiene article, cleaning composition, textile detergent composition, composition for scent dispensers, food, food supplement, pharmaceutical composition or crop protection composition, can be carried out by a method which comprises including a compound (I.1), wherein R¹ and R² are - C(=O)CH₃, in a perfume composition, body care composition, product for oral and dental hygiene, hygiene article, cleaning composition, textile detergent composition, composition for scent dispensers, food, food supplement, pharmaceutical composition or crop protection composition.

Imparting a fruity, woody, sweet note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including a compound (I.2), wherein R¹ and R² are -C(=O)CH₃, in a perfume composition, body care composition, product for oral and dental hygiene, hygiene article, cleaning composition, textile detergent composition, composition for scent dispensers, food, food supplement, pharmaceutical composition or crop protection composition.

Imparting a sweet, fruity note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including a compound (I.4), wherein R¹ and R² are -C(=O)CH₃, in a perfume composition, body care composition, product for oral and dental hygiene, hygiene article, cleaning composition, textile detergent composition, composition for scent dispensers, food, food supplement, pharmaceutical composition or crop protection composition.

Imparting a sweet, red berries note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including an isomer mixture of compounds of formula (I.1), (I.2) and (I.3), where in each case R¹ and R² are -C(=O)CH₂CH₃, in a perfume composition, body care composition, product for oral and dental hygiene, hygiene article, cleaning composition, textile detergent composition, composition for scent dispensers, food, food supplement, pharmaceutical composition or crop protection composition. Specifically, in the mixture, the compounds (I.1), (I.2) and (I.3), where in each case R¹ and R² are - C(=O)CH₂CH₃, are present in a weight ratio of ca. 50:25:25.

Imparting a herbal, algae, green, watery, fruity note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including an isomer mixture of compounds of formula (I.1), (I.2) and (I.3), where in each case R¹ and R² are -CH₃, in a perfume composition, body care composition, product for oral and dental hygiene, hygiene article, cleaning composition, textile detergent composition, composition for scent dispensers, food, food supplement, pharmaceutical composition or crop protection composition. Specifically, in the mixture, the compounds (I.1), (I.2) and (I.3), where in each case R¹ and R² are -CH₃, are present in a weight ratio of ca. 50:25:25.

Imparting a green, herbal, raw potato, green banana note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including an isomer mixture of compounds of formula (I.1), (I.2) and (I.3), where in each case R¹ and R² are -CH₂CH₃, in a perfume composition, body care composition, product for oral and dental hygiene, hygiene article, cleaning composition, textile detergent composition, composition for scent dispensers, food, food supplement, pharmaceutical composition or crop protection composition. Specifically, in the mixture, the compounds (I.1), (I.2) and (I.3), where in each case R¹ and R² are -CH₂CH₃, are present in a weight ratio of ca. 50:25:25.

Imparting a cedar, citrus, warm, herbal note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including an isomer mixture of compounds of formula (I.1), (I.2) and (I.3), where in each case R¹ and R² are -CH₂CH₂CH₂CH₃, in a perfume composition, body care composition, product for oral and dental hygiene, hygiene article, cleaning composition, textile detergent composition, composition for scent dispensers, food, food supplement, pharmaceutical composition or crop protection composition. Specifically, in the mixture, the compounds (I.1), (I.2) and (I.3), where in each case R¹ and R² are -CH₂CH₂CH₂CH₃, are present in a weight ratio of ca. 50:25:25.

Imparting a green, banana, apple (ripe) note to a perfume composition, body care composition, product for oral and dental hygiene, hygiene article, cleaning composition, textile detergent composition, composition for scent dispensers, food, food supplement, pharmaceutical composition or crop protection composition can be carried out by a method which comprises including a compound (I.4), wherein R¹ and R² are -CH₂CH₃, in a perfume composition, body care composition, product for oral and dental hygiene, hygiene article, cleaning composition, textile detergent composition, composition for scent dispensers, food, food supplement, pharmaceutical composition or crop protection composition.

The above "ca." terms take account of measurement errors which may occur in determining the ratio and includes a deviation of ±10%, preferably of ±5%.

Another embodiment of the invention is a composition comprising a mixture of at least two compounds selected from the compounds of formulae (I.1), (I.2) and (I.3), and optionally also (I.4) 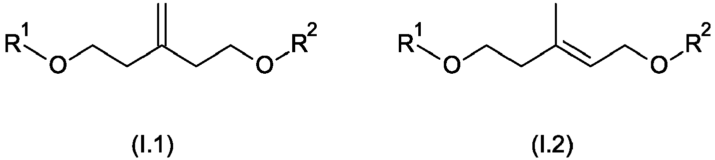 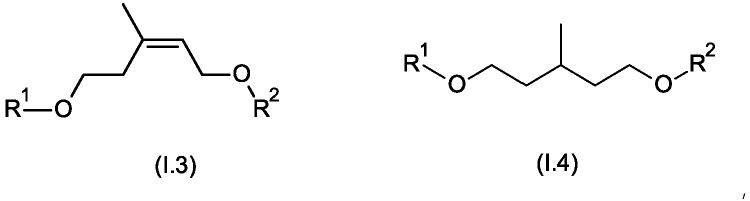
wherein R¹ and R² have one of the meanings as defined above and below, comprising each of the compounds in an amount of 1 - 99 % by weight, with the proviso that weight of the compounds of formulae (I.1), (I.2), (I.3) and (I.4) in said mixture adds up to 100%,
except for following mixtures:
   - mixture of [(E)-5-acetoxy-3-methyl-pent-3-enyl] acetate and [(Z)-5-acetoxy-3-methyl-pent-3-enyl] acetate,
   - mixture of [(E)-3-methyl-5-propanoyloxy-pent-3-enyl] propanoate and [(Z)-3-methyl-5-propanoyloxy-pent-3-enyl] propanoate,
   - mixture of (E)-1,5-dimethoxy-3-methyl-pent-2-ene and (Z)-1,5-dimethoxy-3-methyl-pent-2-ene,
   - mixture of (E)-1,5-diethoxy-3-methyl-pent-2-ene and (Z)-1,5-diethoxy-3-methyl-pent-2-ene,
   - mixture of (E)-3-methyl-1,5-dipropoxy-pent-2-ene and (Z)-3-methyl-1,5-dipropoxy-pent-2-ene, and
   - mixture of (E)-1,5-dibutoxy-3-methyl-pent-2-ene and (Z)-1,5-dibutoxy-3-methyl-pent-2-ene.

The composition might on the one hand consist of said mixture and contain no other component (and thus be the mixture). On the other hand, the composition might contain further components, such as minor amounts of by-products stemming from the production process. If such by-products are present, they amount to at most 10% by weight, preferably at most 5% by weight, more preferably at most 2 %by weight, and specifically at most 1% by weight, relative to the total weight of the compounds of formulae (I.1), (I.2), (I.3) and (I.4) and all by-products.

In a preferred embodiment the invention relates to a mixture of at least two compounds selected from the compounds of formulae (I.1), (I.2) and (I.3), comprising each of the compounds in an amount of 1 - 99 % by weight, with the proviso that the weight of the compounds of formulae (I.1), (I.2) and (I.3) in said mixture adds up to 100%.

Another preferred embodiment relates to a composition comprising such a mixture.

In a particular embodiment, the composition and the mixture contain the compound of formula (I.1) and either one of the compounds of formulae (I.2) and (I.3) or both compounds of formulae (I.2) and (I.3); and in particular contain all three compounds (I.1), (I.2) and (I.3).

More preferably, the mixture contains all three compounds of formulae (I.1), (I.2) and (I.3), comprising each of the compounds in an amount of 1 - 99 % by weight, with the proviso that the weight of the compounds of formulae (I.1), (I.2) and (I.3) in said mixture adds up to 100%, and wherein the ratio of (I.1):(I.2):(I.3) is in the range of 90:5:5 to 10:45:45, preferably 80:10:10 to 20:40:40, especially 55:22.5:22.5 to 45:27.5:27.5, and more especially is approximately 50:25:25. The term "approximately" takes account of measurement errors which may occur in determining the ratio and includes a deviation of ±10%, preferably of ±5%.

Another more preferred embodiment relates to a composition comprising such a mixture.

In a particular embodiment, in the compositions or the mixtures comprising at least two compounds selected from the compounds of formulae (I.1), (I.2) and (I.3), R¹ and R² are C₁-C₄-alkyl.

In another particular embodiment, in the compositions or the mixtures comprising at least two compounds selected from the compounds of formulae (I.1), (I.2) and (I.3), R¹ is -(C=O)-R³, R² -(C=O)-R⁴, and R³ and R⁴ are C₁-C₄-alkyl.

In all compounds of formulae (I.1), (I.2) and (I.3) present in the mixture or composition of the invention all radicals R¹ and R² are identical.

A preferred embodiment of the present invention relates to a composition or a mixture comprising at least two compounds (I) selected from the compounds of formulae (I.1), (I.2) and (I.3), where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of > 0% by weight, based on the total weight of (I.1), (I.2) and (I.3), and that the weight of compound (I.1), (I.2) and (I.3) present in the mixture adds up to 100 %.

A more preferred embodiment of the present invention relates to a composition or a mixture comprising all three compounds of formulae (I.1), (I.2) and (I.3), wherein
- (I.1) is present in an amount of from 25 to 75 weight %, preferably from 30 to 60 weight %, especially from 35 to 55 weight %, based on the total weight of (I.1), (I.2) and (I.3),
- (I.2) is present in an amount of from 15 to 35 weight %, preferably from 18 to 30 weight %, especially from 20 to 28 weight %, based on the total weight of (I.1), (I.2) and (I.3),
- (I.3) is present in an amount of from 15 to 35 weight %, preferably from 18 to 30 weight %, especially from 20 to 28 weight %, based on the total weight of (I.1), (I.2) and (I.3),
with the proviso that the weight of compound (I.1), (I.2) and (I.3) adds up to 100 %.

A special embodiment relates to a composition or a mixture comprising all three compounds of general formulae (I.1), (I.2), and (I.3) comprising each of the compounds in an amount of 1 - 99 % by weight, with the proviso that the weight of the compounds of formulae (I.1), (I.2), and (I.3) adds up to 100%, the ratio of (I.1):(I.2):(I.3) is in the range of 90:5:5 to 10:45:45, preferably 80:10:10 to 20:40:40, especially 55:22.5:22.5 to 45:27.5:27.5, and more especially is approximately 50:25:25. The term "approximately" takes account of measurement errors which may occur in determining the ratio and includes a deviation of ±10%, preferably of ±5%.

A preferred embodiment of the present invention relates to a composition or a mixture comprising at least two ether compounds (I.a) selected from the compounds of formulae (I.1), (I.2) and (I.3) wherein R¹ and R² are as defined in the above first embodiment relating to ether compounds, where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of > 0% by weight, based on the total weight of (I.1), (I.2) and (I.3), and that the weight of compound (I.1), (I.2) and (I.3) present in the mixture adds up to 100 %.

A more preferred embodiment of the present invention relates to a composition or a mixture comprising all three ether compounds selected from the compounds of formulae (I.1), (I.2) and (I.3) wherein R¹ and R² are as defined in the above second embodiment relating to ester compounds, where
- (I.1) is present in an amount of from 25 to 75 weight %, preferably from 30 to 60 weight %, especially from 35 to 55 weight %, based on the total weight of (I.1), (I.2) and (I.3),
- (I.2) is present in an amount of from 15 to 35 weight %, preferably from 18 to 30 weight %, especially from 20 to 28 weight %, based on the total weight of (I.1), (I.2) and (I.3),
- (I.3) is present in an amount of from 15 to 35 weight %, preferably from 18 to 30 weight %, especially from 20 to 28 weight %, based on the total weight of (I.1), (I.2) and (I.3),
with the proviso that the weight of compound (I.1), (I.2) and (I.3) adds up to 100 %.

A special embodiment relates to a composition or a mixture comprising all three ether compounds of the general formulae (I.1), (I.2), and (I.3) comprising each of the compounds in an amount of 1 - 99 % by weight, with the proviso that weight of compound of formulae (I.1), (I.2), and (I.3) adds up to 100%, the ratio of (I.1):(I.2):(I.3) is in the range of 90:5:5 to 10:45:45, preferably 80:10:10 to 20:40:40, especially 55:22.5:22.5 to 45:27.5:27.5, and more especially is approximately 50:25:25. The term "approximately" takes account of measurement errors which may occur in determining the ratio and includes a deviation of ±10%, preferably of ±5%.

A preferred embodiment of the present invention relates to a composition or a mixture comprising at least two ester compounds (I.b) selected from the compounds of formulae (I.1), (I.2) and (I.3) wherein R¹ and R² are as defined in the above second embodiment relating to ester compounds, where
- (I.1) is present in an amount of from 0 to 75 weight %, preferably from 10 to 75 weight %, based on the total weight of (I.1), (I.2) and (I.3),
- (I.2) is present in an amount of from 0 to 50 weight %, preferably from 5 to 45 weight %, based on the total weight of (I.1), (I.2) and (I.3),
- (I.3) is present in an amount of from 0 to 50 weight %, preferably from 0 to 45 weight %, based on the total weight of (I.1), (I.2) and (I.3),
with the proviso that at least two of the compounds (I.1), (I.2) and (I.3) are present in an amount of > 0% by weight, based on the total weight of (I.1), (I.2) and (I.3), and that the weight of compound (I.1), (I.2) and (I.3) present in the mixture adds up to 100 %.

A more preferred embodiment of the present invention relates to a composition or a mixture comprising all three ester compounds selected from the compounds of formulae (I.1), (I.2) and (I.3) wherein R¹ and R² are as defined in the above second embodiment relating to ester compounds, where
- (I.1) is present in an amount of from 25 to 75 weight %, preferably from 30 to 60 weight %, especially from 35 to 55 weight %, based on the total weight of (I.1), (I.2) and (I.3),
- (I.2) is present in an amount of from 15 to 35 weight %, preferably from 18 to 30 weight %, especially from 20 to 28 weight %, based on the total weight of (I.1), (I.2) and (I.3),
- (I.3) is present in an amount of from 15 to 35 weight %, preferably from 18 to 30 weight %, especially from 20 to 28 weight %, based on the total weight of (I.1), (I.2) and (I.3),
with the proviso that the weight of compound (I.1), (I.2) and (I.3) adds up to 100 %.

A further special embodiment relates to a composition or a mixture comprising all three ester compounds of the general formulae (I.1), (I.2), and (I.3) comprising each of the compounds in an amount of 1 - 99 % by weight, with the proviso that weight of compound of formulae (I.1), (I.2), and (I.3) adds up to 100%, the ratio of (I.1):(I.2):(I.3) is in the range of 90:5:5 to 10:45:45, preferably 80:10:10 to 20:40:40, especially 55:22.5:22.5 to 45:27.5:27.5, and more especially is approximately 50:25:25. The term "approximately" takes account of measurement errors which may occur in determining the ratio and includes a deviation of ±10%, preferably of ±5%.

Specifically, the mixture of the invention is selected from the group consisting of following mixtures:
- mixtures of (5-formyloxy-3-methylene-pentyl) formate, [(E)-5-formyloxy-3-methyl-pent-3-enyl] formate and [(Z)-5-formyloxy-3-methyl-pent-3-enyl] formate;
- mixtures of (5-acetoxy-3-methylene-pentyl) acetate, [(E)-5-acetoxy-3-methyl-pent-3-enyl] acetate and [(Z)-5-acetoxy-3-methyl-pent-3-enyl] acetate;
- mixtures of (3-methylene-5-propanoyloxy-pentyl) propanoate, [(E)-3-methyl-5-pro-panoyloxy-pent-3-enyl] propanoate and [(Z)-3-methyl-5-propanoyloxy-pent-3-enyl] propanoate,
- mixtures of 1,5-dimethoxy-3-methylene-pentane, (E)-1,5-dimethoxy-3-methyl-pent-2-ene and (Z)-1,5-dimethoxy-3-methyl-pent-2-ene,
- mixtures of 1,5-diethoxy-3-methylene-pentane, (E)-1,5-diethoxy-3-methyl-pent-2-ene and (Z)-1,5-diethoxy-3-methyl-pent-2-ene, and
- mixtures of 1,5-di-n-butoxy-3-methylene-pentane, (E)-1,5-di-n-butoxy-3-methyl-pent-2-ene and (Z)-1,5-di-n-butoxy-3-methyl-pent-2-ene.

Specifically, the composition of the invention contains one of the above specific mixtures.

More specifically, the mixture of the invention is selected from the group consisting of following mixtures:
- mixture of (5-formyloxy-3-methylene-pentyl) formate, [(E)-5-formyloxy-3-methyl-pent-3-enyl] formate and [(Z)-5-formyloxy-3-methyl-pent-3-enyl] formate in a weight ratio of ca. 50:25:25;
- mixture of (5-acetoxy-3-methylene-pentyl) acetate, [(E)-5-acetoxy-3-methyl-pent-3-enyl] acetate and [(Z)-5-acetoxy-3-methyl-pent-3-enyl] acetate in a weight ratio of ca. 50:25:25;
- mixture of (3-methylene-5-propanoyloxy-pentyl) propanoate, [(E)-3-methyl-5-propa-noyloxy-pent-3-enyl] propanoate and [(Z)-3-methyl-5-propanoyloxy-pent-3-enyl] propanoate in a weight ratio of ca. 50:25:25,
- mixture of 1,5-dimethoxy-3-methylene-pentane, (E)-1,5-dimethoxy-3-methyl-pent-2-ene and (Z)-1,5-dimethoxy-3-methyl-pent-2-ene in a weight ratio of ca. 50:25:25,
- mixture of 1,5-diethoxy-3-methylene-pentane, (E)-1,5-diethoxy-3-methyl-pent-2-ene and (Z)-1,5-diethoxy-3-methyl-pent-2-ene in a weight ratio of ca. 50:25:25, and
- mixture of 1,5-di-n-butoxy-3-methylene-pentane, (E)-1,5-di-n-butoxy-3-methyl-pent-2-ene and (Z)-1,5-di-n-butoxy-3-methyl-pent-2-ene in a weight ratio of ca. 50:25:25.

The above "ca." terms take account of measurement errors which may occur in determining the ratio and includes a deviation of ±10%, preferably of ±5%.

More specifically, the composition of the invention contains one of the above more specific mixtures.

Preparing the compound of formula (I), a stereoisomer thereof or a mixture of stereoisomers as defined above can be carried out by a method comprising the following steps:
(i) reacting 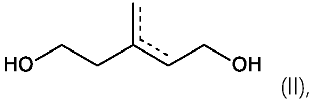 with 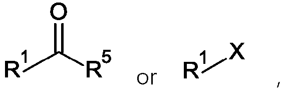 wherein
   one of the bonds depicted as -̅ -̅ is a single bond and the other is a double bond or a single bond,
   - R¹: has one of the meanings given above,
   - R²: has one of the meanings given above,
   - R⁵: is X or -O-C(=O)-R², and
   - X: represents a leaving group,
   to obtain a raw-product mixture, and
(ii) optionally subjecting the obtained raw-product mixture in step (i) to a purification step.

Accordingly, preparing the compound of formula (I.a), a stereoisomer thereof or a mixture of stereoisomers thereof as defined above can be carried out by a method comprising
(i) reacting compound (II) or mixtures thereof 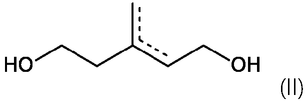 with the alkylation reagent R^{1a}-X or R^{2a}-X, wherein R^{1a} and R^{2a} have one of the meanings given above and X represents a leaving group, selected from halogen, such as Cl, Br, I, and sulfonates, such as tosylate, mesylate, triflate or nonaflate, in the presence of a base to obtain a raw-product mixture, and
(ii) subjecting the raw-product mixture obtained in step (i) to a purification step.

The alkylation reaction in step (i) is performed under conventional alkylation reaction conditions that are well known to the skilled person.

Suitable bases that can be used in the alkylation reaction are as defined above.

The preparation of diether compounds of the general formula (I.a), where the radicals R^{1a} and R^{2a} are identical, is typically performed by reacting compound (II) or mixtures thereof with at least two equivalents, e.g. 2.0, 2.1, 2.5 or 3.0 equivalents, of the alkylation reagent R^{1a}-X or R^{2a}-X, respectively, wherein the radicals R^{1a}, R^{2a} and X have one of the meanings given above, and the obtained raw product is subsequently subjected to a purification step, as defined above.

Generally, the starting material compound (II) or mixtures thereof is applied as exo/E/Z/sat isomer mixture, in particular exo/E/Z isomer. Consequently, the compounds (I.a) are typically obtained as a mixture of exo/E/Z/sat isomers, in particular exo/E/Z isomer.

In step (ii) of the method for preparing the compound of formula (I.a), the raw-product mixture obtained in step (i) is subjected to a purification step, in order to remove unwanted by-products or impurities, such as residual mono-substituted compound (I.a-OH) and, if desired, to separate the exo/E/Z isomers.

Generally the purification in step (ii) can be performed by using common purification methods, such as crystallization, distillation or chromatographic methods, e.g. column chromatography or high performance liquid chromatography. In case where only minor amounts of impurities and by-products are present in the raw-product mixture the purification step can also be performed via a simple extractive workup. If desired the cis/trans isomers can be separated by fractional crystallization, column chromatography or high performance liquid chromatography.

Accordingly, preparing the compound of formula (I.b), a stereoisomer thereof, or a mixture of stereoisomers, as defined above can be carried out by a method comprising
(i) reacting compound (II) or mixtures thereof 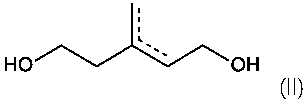
   with an acylating agent 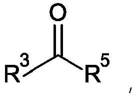 or with an acylating agent
   wherein R⁵ is OH, X or -O-C(=O)-R³, wherein X represents a leaving group, selected from halogen, such as Cl, Br, or I, in particular Cl or Br, and R³ and R⁴ have one of the meanings as defined above, in particular reacting compound (II) or mixtures thereof with a carboxylic acid R³-COOH or an anhydride thereof, or with the acid halide R³-(C=O)X' in the presence of an organic base, or with the carboxylic acid R⁴-COOH or an anhydride thereof, or with the acid halogenide R⁴-(C=O)X', wherein R³ and R⁴ have one of the meanings given above and X' represents a halogen atom, such as Cl, Br or I, in particular Cl or Br, to obtain a raw-product mixture, and
(ii) optionally subjecting the raw-product mixture obtained in step (i) to a purification step.

The individual reaction conditions for the preparation of the ester compounds of the general formula (I.b) in step i) are well known to the skilled person.

The preparation of diester compounds of the general formula (I.b), where the radicals R^{1b} and R^{2b} are identical, is typically performed by reacting compound (II) or mixtures thereof with at least two equivalents, e.g. 2.0, 2.1, 2.5 or 3.0 equivalents, of an acylating agent, e.g. a carboxylic acid R³-COOH or an anhydride thereof, or an acid halide R³-(C=O)X' in the presence of an organic base or with the carboxylic acid R⁴-COOH or an anhydride thereof or with the acid halide R⁴-(C=O)X', wherein R³ and R⁴ have one of the meanings given above and X' represents a halogen atom, such as Cl, Br or I, in particular Cl or Br, to obtain a raw-product mixture, and, if necessary, the obtained raw product is subsequently subjected to a purification step, as defined above.

In step (ii) of the method for preparing the compound of formula (I.b), if necessary, the raw-product mixture obtained in step (i) is subjected to a purification step, in order to remove unwanted by-products or impurities, such as residual mono-substituted compound (I.b-OH) and, if desired, to separate the exo/E/Z isomers.

Generally the purification in step (ii) can be performed by using common purification methods, such as crystallization, distillation or chromatographic methods, e.g. column chromatography or high performance liquid chromatography. In case where only minor amounts of impurities and by-products are present in the raw-product mixture the purification step can also be performed via a simple extractive workup. If desired the exo/Z/E isomers can be separated by fractional crystallization, column chromatography or high performance liquid chromatography.

The starting material compound (II) can be a pure compound (II.1), (II.2), (II.3) or (II.4) or a mixture of different stereoisomers [E/Z mixture; E = (II.2); Z =(II.3)] or of double bond position isomers [mixture of (II.1) = exo with (II.2) and/or (II.3)] or a mixture of saturated and unsaturated compounds [mixture of [II.4] with at least one of (II.1), (II.2) and (II.3)].

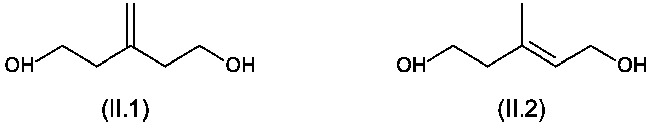

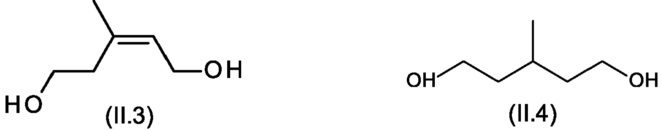

A by-product in the citral production process is an exo/E/Z isomer mixture of II (mixture of [II.1), (II.2) and (II.3)], which is a suitable starting material for the preparation of compounds (I). Consequently, the compounds I.b are typically obtained as a mixture of exo/E/Z isomers when this mixture is used.

The present invention is now illustrated in further detail by the following examples, without imposing any limitation thereto.

### EXAMPLES

### Abbreviations:

- DMAP:: 4-(N,N'-dimethylamino)-pyridine
- MTBE:: 2-methoxy-2-methylpropane (methyl-tert-butylether)
- RT:: room temperature (20-25°C)
- THF:: tetrahydrofuran

Citral s.s. diol: mixture of 3-methylenepentane-1,5-diol, (E)-3-methylpent-2-ene-1,5-diol and (Z)-3-methylpent-2-ene-1,5-diol (available as a side stream from the citral production)

### Analytics:

The purity of the products was determined by Gas Chromatography area-%:
GC-system: Agilent 7890 B;
GC-Column: DB-WAX (30 m (length), 0.32 mm (ID), 0.25 micrometer (film));
Temperature program: 85°C to 230°C at 5°/min

### A. Preparation

### Example 1:

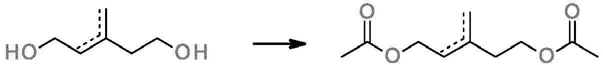

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, | 116.16 | 20g | 0.172 |
| molar ratio exo:E:Z is 50:25:25 | | | |
| acetic anhydride | 102.09 | 39.5g | 0.387 |
| 4-(N,N'-dimethylamino)-pyridine | 122.17 | 0.631g | 0.005 |
| tetrahydrofuran | | 150mL | |

DMAP was added to the THF solution of the citral s.s. diol mixture at RT. The mixture was set to reflux (53°C) and acetic anhydride (2.25 eq) was slowly added at this temperature. After 1 h, 99% diol conversion was observed by GC. The reaction was cooled down to room temperature and slowly quenched with 100 mL of water. 100 mL of ethyl acetate were then added. The organic phase was separated and washed with NaHCO₃ and brine solution. The organic extracts were combined and dried with sodium sulfate. After evaporating the solvent at reduced pressure, 37.1 g of crude product that contained ca. 90% of the diacetate according to the GC (area %) were obtained. The product was purified by distillation. The mixture of the corresponding di-acetates was obtained with a purity of 93.2% (GC area %). The NMR confirmed that the composition of the mixture was 50% of the 3-methylene di-acetate and 50% of a 1:1 E/Z mixture of the 2-pentene derivatives.

| | Atom number | ¹³C shifts measured (multiplicity) |
|---|---|---|
| 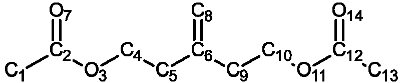 | C1/ C13 | 20.95 (q) |
| | C2/ C12 | 170.01 (s) |
| | C4/ C10 | 62.59 (t) |
| | C5/ C9 | 35.01 (t) |
| | C6 | 141.54 (s) |
| | C8 | 113.53 (t) |

| | Atom number | ¹³C shifts measured (multiplicity) |
|---|---|---|
| 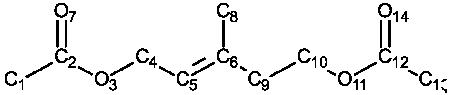 | C1/C13 | 21.02/ 20.95 (q) |
| | C2/ C12 | 170.99 (s) |
| | C4 | 62.28 (t) |
| | C5 | 120.91 (d) |
| | C6 | 137.90 (s) |
| | C8 | 16.51 (q) |
| | C9 | 38.38 (t) |
| | C10 | 61.06 (t) |

| | Atom number | ¹³C shifts measured (multiplicity) |
|---|---|---|
| 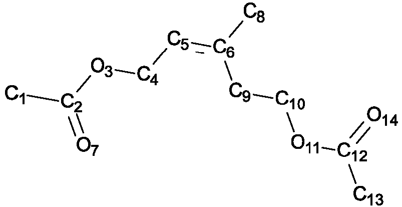 | C1/ C13 | 21.02/ 20.95 (q) |
| | C2/ C12 | 171.01/ 170.99 (s) |
| | C4 | 62.36 (t) |
| | C5 | 121.86 (d) |
| | C6 | 138.07 (s) |
| | C8 | 23.63 (q) |
| | C9 | 31.35 (t) |
| | C10 | 60.85 (t) |

### Example 2:

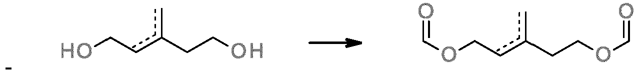

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, | 116.16 | 20g | 0.172 |
| molar ratio exo:E:Z: 50:25:25 | | | |
| formic acid | 46.03 | 55.48q | 1.205 |
| acetic anhydride | 102.09 | 105.47g | 1.033 |
| 4-(N,N'-dimethylamino)-pyridine | 122.17 | 0.421 g | 0,02 |
| tetrahydrofuran | | 100mL | |

At 0°C formic acid was slowly added to acetic anhydride. The mixture was stirred 1.5 h at 55°C. The mixture was then cooled down to 0°C and 50 mL of THF were added. At this temperature a solution of the citral s.s. diol mixture in 50 mL THF was slowly added, followed by the addition of DMAP to the mixture. The reaction was stirred at room temperature for 4h and full conversion was observed by GC. 100 mL of toluene were added to the mixture and the organic phase was washed 3 times with 30 mL of water. The organic phase was dried with sodium sulfate and the solvent was removed in the rotatory evaporator to give 19.6 g of crude product that contained ca 90% of the diformate according to the GC (area %). The product was purified by distillation. The mixture of the corresponding di-formates was obtained with a purity of 98.1% (GC area %). The NMR confirmed that the composition of the mixture was 50% of the 3-methylene diformate and 50% of a 1:1 E/Z mixture of the 2-pentene derivatives.

| | Atom number | ¹³C shifts measured (multiplicity) |
|---|---|---|
| 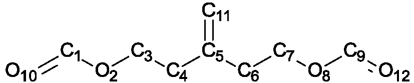 | C1/ C9 | 160.95 (d) |
| | C3/ C7 | 61.91 (t) |
| | C4/ C6 | 34.81 (t) |
| | C5 | 140.87 (s) |
| | C11 | 114.04 (t) |

| | Atom number | ¹³C shifts measured (multiplicity) |
|---|---|---|
| 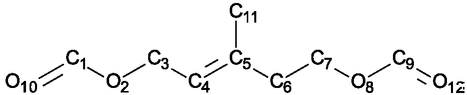 E:Z. Mixture 1:1 | C1/ C9 | 160.97/ 160.89 (s) |
| | C3 | 60.33/ 60.10 (t) |
| | C4 | 121.57/ 121.00 (s) |
| | C5 | 138.45 (s) |
| | C6 | 38.19/ 31.21 (t) |
| | C7 | 61.64/ 61.59 (d) |
| | C11 | 23.44/ 16.43 (q) |

### Example 3:

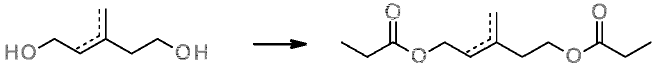

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, | 144.21 | 20g | 0.172 |
| molar ratio exo:E:Z: 50:25:25 | | | |
| propionyl chloride | 92.52 | 39.83g | 0.430 |
| 4-(N,N'-dimethylamino)-pyridine | 122.17 | 0.300g | 0.01 |
| triethylamine | 101.19 | 43.56g | 0.430 |
| hexane | | 100mL | |

A solution of the citral s.s. diol mixture, triethylamine and DMAP in hexane was cooled down to 0°C. At this temperature, propionyl chloride was slowly added. The reaction was stirred at room temperature for 1h, 50 mL of hexane were added and the reaction was stirred 4 h at reflux (67°C). After this time full conversion was observed by GC. The reaction was cooled down to room temperature and slowly quenched with 100 mL of water. 100 mL of ethyl acetate were added, and the organic phase was washed with NaHCO₃ and brine solution. The organic extracts were combined and dried with sodium sulfate. After evaporating the solvent at reduced pressure, 38.4 g of crude product that contained ca. 90% of the dipropionate according to the GC (area %) were obtained. The product was purified by distillation. The mixture of the corresponding di-propionates was obtained with a purity of 97.8% (GC area %). The NMR confirmed that the composition of the mixture was 50% of the 3-methylene di-propionate and 50% of a 1:1 E/Z mixture of the 2-pentene derivatives.

| | Atom number | ¹³C shifts measured (multiplicity) |
|---|---|---|
| 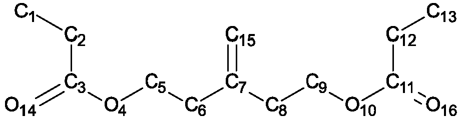 | C1/ C13 | 9.13 (q) |
| | C2/ C12 | 27.56 (t) |
| | C3/ C11 | 174.41 (s) |
| | C5/ C9 | 62.43 (t) |
| | C6/ C8 | 35.07 (t) |
| | C7 | 141.61 (s) |
| | C15 | 113.54 (t) |

| | Atom number | ¹³C shifts measured (multiplicity) |
|---|---|---|
| 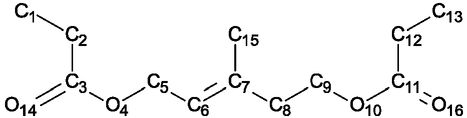 E:Z Mixture 1:1 | C1/ C13 | 9.14/ 9.08 (q) |
| | C2/ C12 | 27.56 (t) |
| | C3/ C11 | 174.41 (s) |
| | C5/ C9 | 62.20/ 62.19/ 60.95/ 60.77(t) |
| | C6 | 121.93/ 121.04 (d) |
| | C7 | 138.01/ 137.83 (s) |
| | C8 | 38.46/ 31.40 (t) |
| | C15 | 23.66/ 16.50 (q) |

### Example 4:

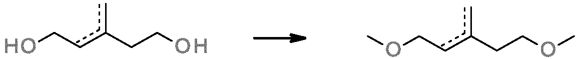

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, | 116.16 | 15g | 0.129 |
| molar ratio exo:E:Z: 50:25:25 | | | |
| methyl iodide | 141.94 | 42.16g | 0.297 |
| sodium hydride | 23.99 | 7.12g | 0.297 |
| tetrahydrofuran | | 105mL | |

To a dispersion of sodium hydride (2.3 eq) in 75 mL of THF a solution of the citral s.s. diol mixture in 30 mL of THF was slowly added at 0°C. The mixture was stirred 30 min at 0°C. Then 2.3 eq of methyl iodide were slowly added at RT and the mixture was subsequently stirred at 40°C for 4 h. The reaction mixture was cooled down to 0°C, and the addition of 0.75 eq of NaH followed by 0.75 eq of methyl iodide was repeated. The mixture was then stirred for 20h at 40°C. At this point, the reaction was slowly quenched with 50 mL of water. The organic phase was extracted with 3 times 50 mL MTBE. The organic extracts were combined and washed with 50 mL of NH₃ solution and with 50mL of brine solution. The organic extracts were combined and dried with sodium sulfate. After evaporating the solvent at reduced pressure 20 g of crude product were obtained with 98% of the diether according to the GC (area %). The product was purified by distillation. The mixture of the corresponding di-methyl ethers was obtained with a purity > 99% (GC area %). The NMR confirmed that the composition of the mixture was 50% of the exo-di-methyl ether and 50% of a 1:1 E/Z mixture of the 2-pentene derivative.

| | Atom number | ¹³C shifts measured (multiplicity) |
|---|---|---|
| 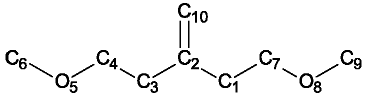 | C1/ C3 | 36.11 (t) |
| | C4/ C7 | 71.08 (t) |
| | C6/ C9 | 58.56 (q) |
| | C2 | 143.52 (s) |
| | C10 | 112.07 (t) |

| | Atom number | ¹³C shifts measured (multiplicity) |
|---|---|---|
| 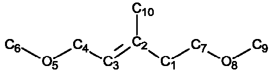 | C1 | 39.40/ 32.49 (t) |
| | C2 | 137.27/ 137.23 (s) |
| | C3 | 123.47/ 122.42 (d) |
| E:Z Mixture 1:1 | C4/C7 | 71.14/ 71.06/ 68.83/ 68.65 (t) |
| | C6/C9 | 57.91/ 57.87 (q) |
| | C10 | 23.82/ 16.61 (q) |

### Example 5:

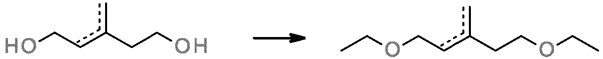

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, molar ratio exo:E:Z: 50:25:25 | 116.16 | 15g | 0.129 |
| ethyl iodide | 155.97 | 46.32g | 0.297 |
| sodium hydride | 23.99 | 7.12g | 0.297 |
| tetrahydrofuran | | 105mL | |

To a dispersion of sodium hydride (2.3 eq) in 75 mL of THF a solution of the citral s.s. diol mixture in 30 mL of THF was slowly added at 0°C. The mixture was stirred 30 min at 0°C. Then 2.3 eq of ethyl iodide were slowly added at RT. After the addition, the mixture was stirred at 40°C for 4 h. The reaction mixture was cooled down to 0°C and the addition of 0.75 eq of NaH followed by 0.75 eq of ethyl iodide was repeated. The mixture was then stirred for 20 h at 40°C. At this point, the reaction was slowly quenched with 50 mL of water. The organic phase was extracted with 3 times 50 mL MTBE. The organic extracts were combined and washed with 50mL of NH₃ solution and with 50mL of brine solution. The organic extracts were combined and dried with sodium sulfate. After evaporating the solvent at reduced pressure 24.6 g of crude product were obtained with 93% of the diether according to the GC (area %). The product was purified by distillation. The mixture of the corresponding diethyl ethers was obtained with a purity > 96% (GC area %). The NMR confirmed that the composition of the mixture was 50% of the exo-diethyl ether and 50% of a 1:1 E/Z mixture of the 2-pentene derivative.

| | Atom number | ¹³C shifts measured (multiplicity) |
|---|---|---|
| 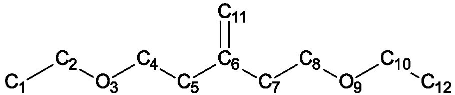 | C1/ C12 | 15.20 (q) |
| | C2/ C10 | 66.16 (t) |
| | C4/ C8 | 69.19 (t) |
| | C5/ C7 | 36.46 (t) |
| | C6 | 143.72 (s) |
| | C11 | 111.84 (t) |

| | Atom number | ¹³C shifts measured (multiplicity) |
|---|---|---|
| 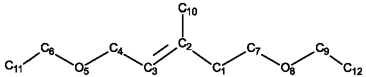 | C1 | 39.65/ 32.80(t) |
| | C2 | 136.87/ 136.79 (s) |
| | C3 | 123.81/ 122.79 (d) |
| E:Z Mixture 1:1 | C4/C7 | 69.53/ 69.30/ 67.01/ 66.85 (t) |
| | C6/C9 | 65.52 (t) |
| | C10 | 24.01/ 16.74 (q) |
| | C11/C12 | 15.24 (q) |

### Example 6:

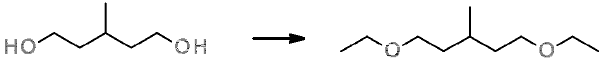

| **Compound** | **MW** | **Mass/Volume** | **Moles** |
|---|---|---|---|
| 3-methyl-1,5-pentanediol | 118.17 | 10g | 0.085 |
| ethyl iodide | 155.97 | 30.35g | 0.195 |
| sodium hydride | 23.99 | 4.67g | 0.195 |
| tetrahydrofuran | | 105mL | |

To a dispersion of sodium hydride (2.3 eq) in 75 mL of THF a solution of 3-methyl-1,5-pentanediol in 30 mL of THF was slowly added at 0°C. The mixture was stirred 30 min at 0°C. Then 2.3 eq of ethyl iodide were slowly added at RT. After the addition, the mixture was stirred at 40°C for 4 h. The reaction mixture was cooled down to 0°C and the addition of 0.75 eq of NaH followed by 0.75 eq of ethyl iodide was repeated. The mixture was then stirred for 20 h at 40°C. At this point, the reaction was slowly quenched with 50 mL of water. The organic phase was extracted with 3 times 50 mL MTBE. The organic extracts were combined and washed with 50 mL of NH₃ solution and with 50 mL of brine solution. The organic extracts were combined and dried with sodium sulfate. After evaporating the solvent at reduced pressure 15.7 g of crude product were obtained with 92% of the diether according to the GC (area %). The product was purified by distillation. The corresponding diethyl ether was obtained with a purity > 97% (GC area %).

| | Atom number | ¹³C shifts measured (multiplicity) |
|---|---|---|
| 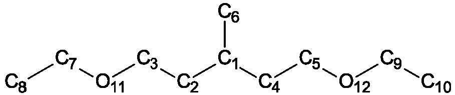 | C1 | 27.21 (d) |
| | C2/ C4 | 36.89 (t) |
| | C3/ C5 | 68.82 (t) |
| | C6 | 19.75 (q) |
| | C7/ C9 | 66.11 (t) |
| | C8/ C10 | 15.27 (q) |

### Example 7:

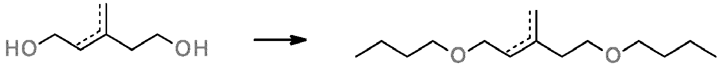

| **Compound** | **MW** | **Mass/Volume** | **Moles** |
|---|---|---|---|
| mixture of 3-methylene-pentane-1,5-diol, (E)-3-methylpent-2-ene-1,5-diol and (Z)-3-methylpent-2-ene-1,5-diol, molar ratio exo:E:Z: 50:25:25 | 116.16 | 15g | 0.129 |
| 1-iodo-butan | 184.02 | 56.65g | 0.297 |
| sodium hydride | 23.99 | 7.12g | 0.297 |
| tetrahydrofuran | | 105mL | |

To a dispersion of sodium hydride (2.3 eq) in 75 mL of THF a solution of the citral s.s. diol mixture in 30 mL of THF was slowly added at 0°C. The mixture was stirred 30 min at 0°C. Then 2.3 eq of ethyl iodide were slowly added at RT. After the addition, the mixture was stirred at 50°C for 4 h. The reaction mixture was cooled down to 0°C and the addition of 0.75 eq of NaH followed by 0.75 eq of 1-iodo-butan was repeated. The mixture was then stirred for 90 h at 50°C. At this point, the reaction was slowly quenched with 50 mL of water. The organic phase was extracted with 3 times 50 mL MTBE. The organic extracts were combined and washed with 50 mL of NH₃ solution and with 50 mL of brine solution. The organic extracts were combined and dried with sodium sulfate. After evaporating the solvent at reduced pressure 26.2 g of crude product were obtained with 37% of the di-n-butyl ether according to the GC (area %), the rest being the mono-ether derivatives. The product was purified by column chromatography. The mixture of the corresponding diethyl ethers was obtained with a purity > 95% (GC area %). The NMR confirmed that the composition of the mixture was 50% of the exo-di-n-butyl ether and 50% of a 1:1 E/Z mixture of the 2-pentene derivative.

| | Atom number | ¹³C shifts measured (multiplicity) |
|---|---|---|
| 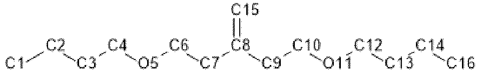 | C1/ C16 | 13.96 (q) |
| | C2/ C14 | 19.44 (t) |
| | C3/ C13 | 31.94 (t) |
| | C4/ C12 | 70.69 (t) |
| | C6/ C10 | 69.50 (t) |
| | C7/ C9 | 36.49 (t) |
| | C8 | 143.92 (s) |
| | C15 | 111.80 (t) |

| 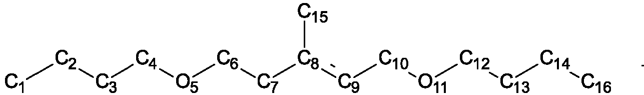 | Atom number | ¹³C shifts measured (multiplicity) |
|---|---|---|
| E:Z Mixture 1:1 | C1/ C16 | 13.96 (q) |
| | C2/ C14 | 19.45/ 19.41 (t) |
| | C3/ C13 | 32.75/ 31.95 (t) |
| | C4/ C12 | 70.14/ 70.05 (t) |
| | C6 | 69.40/ 69.33 (t) |
| | C7 | 39.58 (t) |
| | C8 | 136.98/ 136.89 (s) |
| | C9 | 123.72/ 122.74 (d) |
| | C10 | 67.17/ 67.02 (t) |
| | C15 | 16.77/ 24.03 (q) |

### B. Olfactory assessment

In order to test the quality and intensity of the odor of the compounds obtained in the preparation examples, scent strip tests were performed. For this purpose, strips of absorbent paper were dipped into a solution containing 1 to 10 % by weight solution of the compound to be tested in ethanol. After evaporation of the solvent (about 30 sec.) the scent impression was olfactively evaluated by a trained perfumer. The results are summarized in Table 1.

**Table 1: Results of the scent tests**

| Compound | Odor description |
|---|---|
| 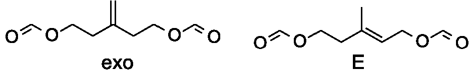 | Technical, mushroom |
| 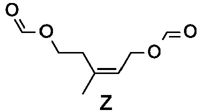 ratio exo:E:Z 50:25:25 | |
| 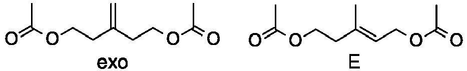 | Sweet, powdery, terpenic, fruity |
| 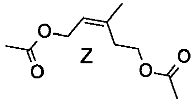 ratio exo:E:Z 50:25:25 | |
| 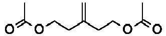 | Fruity, sweet, rubber, musty, dusty |
| 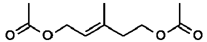 | Fruity, woody, sweet |
| 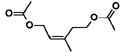 | Weak |
| 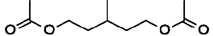 | Sweet, fruity |
| 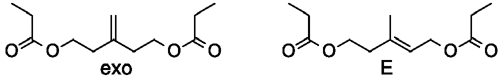 | Sweet, red berries |
| 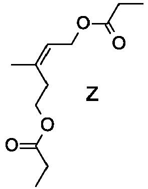 ratio exo:E:Z 50:25:25 | |
| 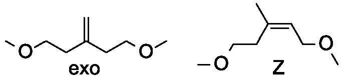 | Herbal, algae, green, watery, fruity |
| 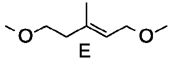 ratio exo:E:Z 50:25:25 | |
| 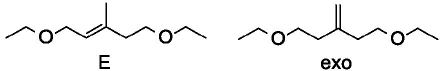 | Green, herbal, raw potato, green banana |
| 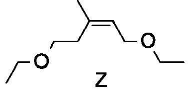 ratio exo:E:Z 50:25:25 | |
| 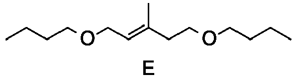 | Cedar, citrus, warm, herbal |
| 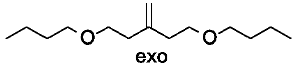 | |
| 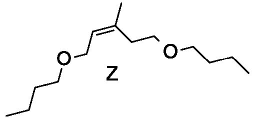 ratio exo:E:Z 50:25:25 | |
| 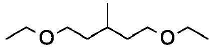 | Green, banana, apple (ripe) |

## Claims

1. The use of a compound of formula (I) 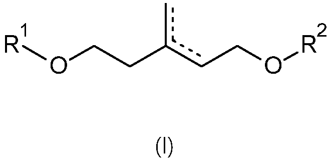 wherein
one of the symbols -̅ -̅ designates a single bond and the other designates a double bond or a single bond, and
R¹ and R² are identical and are either both C₁-C₄-alkyl, or R¹ is -(C=O)-R³ and R² is -(C=O)-R⁴, where R³ and R⁴ are identical and are hydrogen or C₁-C₄-alkyl, or of a stereoisomer thereof, or of a mixture of stereoisomers thereof, or of a mixture of different compounds of formula (I)
as an aroma chemical.

2. The use according to claim 1, where in formula (I)
R¹ is C₁-C₄-alkyl,
R² is C₁-C₄-alkyl.

3. The use according to claim 1 or 2, where the compound of formula (I) has at least one of the following features b) or c)
b) R¹ and R² are C₁-C₃-alkyl,
c) R¹ and R² are methyl or ethyl, preferably methyl.

4. The use according to claim 1, where in formula (I)
R¹ is -(C=O)-R³, and
R² is -(C=O)-R⁴.

5. The use according to claim 4, where R³ and R⁴ are hydrogen or C₁-C₃-alkyl, preferably hydrogen, methyl or ethyl.

6. The use according to any of the preceding claims, of a mixture containing two or three isomers of a compound of formula I, where the isomers are of the formulae (I.1), (I.2) and (I.3),

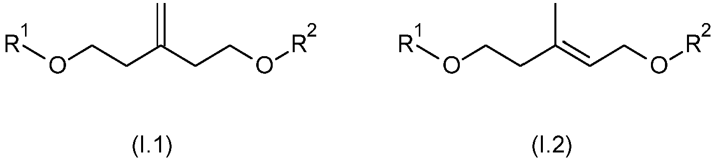

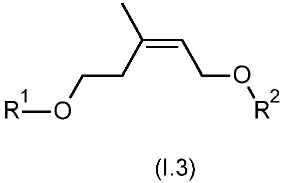

7. The use according to any of the preceding claims, for imparting an olfactory impression, preferably as a fragrance.

8. The use of a compound of formula (I) or of a stereoisomer thereof or of a mixture of stereoisomers thereof or of a mixture of different compounds of formula (I) as defined in any of the preceding claims, for modifying the scent character of a fragranced composition, in particular of a fragranced ready-to-use composition.

9. The use according to any of the preceding claims, in a composition selected from the group consisting of perfume compositions, body care compositions, products for oral and dental hygiene, hygiene articles, cleaning compositions, textile detergent compositions, compositions for scent dispensers, foods, food supplements, pharmaceutical compositions and crop protection compositions.

10. Composition comprising at least one compound of formula (I), a stereoisomer thereof or a mixture of stereoisomers thereof, as defined in any of claims 1 to 6, and at least one further component selected from the group consisting of aroma chemicals different from compounds (I) and non-aroma chemical carriers; where the carrier materials are liquid or oil-like, or solid carrier materials; where the liquid or oil-like carrier materials are selected from methanol, ethanol, ethylene glycol, glycerol, diglycerol, propylene glycol, dipropylene glycol, 1,2-butylene glycol, hexamethylcyclotrisiloxane, decamethylcyclopentasiloxane, diethylene glycol monoethyl ether, diethyl phthalate, isopropyl myristate, triethyl citrate, benzyl benzoate, fractionated coconut oil, tetradecyl acetate, tetradecyl lactate, esters of glycerol with melting temperatures below 20°C, and isopropyl myristate.

11. The composition according to claim 10, selected from the group consisting of perfume compositions, body care compositions, products for oral and dental hygiene, hygiene articles, cleaning compositions, textile detergent compositions, compositions for scent dispensers, foods, food supplements, pharmaceutical compositions and crop protection compositions.

12. A composition comprising a mixture of at least two compounds selected from the group consisting of isomers of formulae (I.1), (I.2) and (I.3), 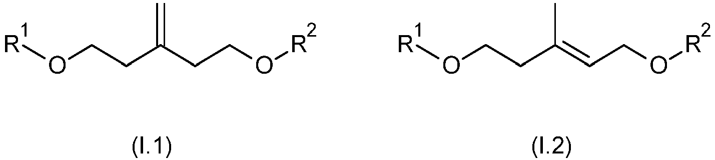 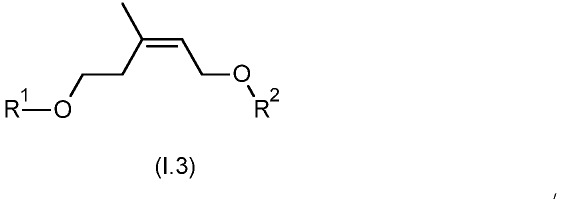
where in all compounds of formulae (I.1), (I.2) and (I.3) present in the mixture all radicals R¹ and R² are identical and are either C₁-C₄-alkyl, or all radicals R¹ are -(C=O)-R³, all radicals R² are -(C=O)-R⁴, and all radicals R³ and R⁴ are identical and are hydrogen or C₁-C₄-alkyl, preferably hydrogen, methyl or ethyl;
comprising each of the compounds present in the mixture in an amount of from 1 - 99 % by weight, with the proviso that the weight of the compounds of formulae (I.1), (I.2) and (I.3) present in said mixture adds up to 100%;
except for following mixtures:
- mixture of [(E)-5-acetoxy-3-methyl-pent-3-enyl] acetate and [(Z)-5-acetoxy-3-methyl-pent-3-enyl] acetate,
- mixture of [(E)-3-methyl-5-propanoyloxy-pent-3-enyl] propanoate and [(Z)-3-methyl-5-propanoyloxy-pent-3-enyl] propanoate,
- mixture of (E)-1,5-dimethoxy-3-methyl-pent-2-ene and (Z)-1,5-dimethoxy-3-methyl-pent-2-ene,
- mixture of (E)-1,5-diethoxy-3-methyl-pent-2-ene and (Z)-1,5-diethoxy-3-methyl-pent-2-ene,
- mixture of (E)-3-methyl-1,5-dipropoxy-pent-2-ene and (Z)-3-methyl-1,5-dipropoxy-pent-2-ene, and
- mixture of (E)-1,5-dibutoxy-3-methyl-pent-2-ene and (Z)-1,5-dibutoxy-3-methyl-pent-2-ene.

13. The composition according to claim 12, containing the compound of formula (I.1) and either one of the compounds of formulae (I.2) and (I.3) or both compounds of formulae (I.2) and (I.3); and in particular containing all three compounds (I.1), (I.2) and (I.3).

14. The composition according to any of claims 12 to 13, comprising a mixture which is selected from the group consisting of
- mixtures of (5-formyloxy-3-methylene-pentyl) formate, [(E)-5-formyloxy-3-methyl-pent-3-enyl] formate and [(Z)-5-formyloxy-3-methyl-pent-3-enyl] formate;
- mixtures of (5-acetoxy-3-methylene-pentyl) acetate, [(E)-5-acetoxy-3-methyl-pent-3-enyl] acetate and [(Z)-5-acetoxy-3-methyl-pent-3-enyl] acetate;
- mixtures of (3-methylene-5-propanoyloxy-pentyl) propanoate, [(E)-3-methyl-5-propanoyloxy-pent-3-enyl] propanoate and [(Z)-3-methyl-5-propanoyloxy-pent-3-enyl] propanoate,
- mixtures of 1,5-dimethoxy-3-methylene-pentane, (E)-1,5-dimethoxy-3-methyl-pent-2-ene and (Z)-1,5-dimethoxy-3-methyl-pent-2-ene,
- mixtures of 1,5-diethoxy-3-methylene-pentane, (E)-1,5-diethoxy-3-methyl-pent-2-ene and (Z)-1,5-diethoxy-3-methyl-pent-2-ene, and
- mixtures of 1,5-di-n-butoxy-3-methylene-pentane, (E)-1,5-di-n-butoxy-3-methyl-pent-2-ene and (Z)-1,5-di-n-butoxy-3-methyl-pent-2-ene.

## Patentansprüche

1. Verwendung einer Verbindung der Formel (I) 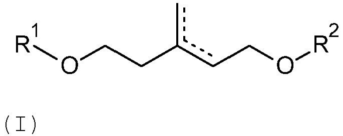 wobei
eines der Symbole -̅ -̅ eine Einfachbindung darstellt und das andere eine Doppelbindung oder eine Einfachbindung darstellt und
R¹ und R² gleich sind und entweder beide für C₁-C₄-Alkyl stehen oder R¹ für -(C=O)-R³ steht und R² für -(C=O)-R⁴ steht, wobei R³ und R⁴ gleich sind und für Wasserstoff oder C₁-C₄-Alkyl stehen, oder eines Stereoisomers davon oder eines Gemischs von Stereoisomeren davon oder eines Gemischs von verschiedenen Verbindungen der Formel (I) als Aromachemikalie.

2. Verwendung nach Anspruch 1, wobei in Formel (I)
R¹ für C₁-C₄-Alkyl steht,
R² für C₁-C₄-Alkyl steht.

3. Verwendung nach Anspruch 1 oder 2, wobei die Verbindung der Formel (I) mindestens eines der folgenden Merkmale b) oder c) aufweist:
b) R¹ und R² stehen für C₁-C₃-Alkyl,
c) R¹ und R² stehen für Methyl oder Ethyl, vorzugsweise Methyl.

4. Verwendung nach Anspruch 1, wobei in Formel (I)
R¹ für -(C=O)-R³ steht und
R² für -(C=O)-R⁴ steht.

5. Verwendung nach Anspruch 4, wobei R³ und R⁴ für Wasserstoff oder C₁-C₃-Alkyl, vorzugsweise Wasserstoff, Methyl oder Ethyl, stehen.

6. Verwendung nach einem der vorhergehenden Ansprüche, eines Gemischs, das zwei oder drei Isomere einer Verbindung der Formel I enthält, wobei die Isomere die Formeln (1.1), (I.2) und (I.3) aufweisen,

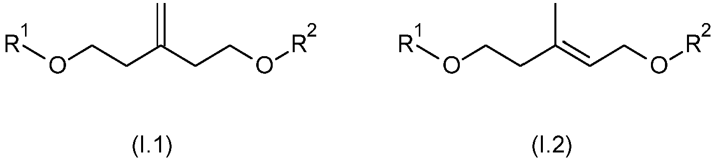

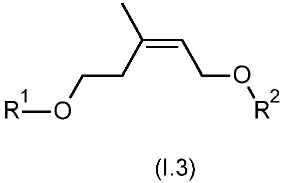

7. Verwendung nach einem der vorhergehenden Ansprüche zum Verleihen eines olfaktorischen Eindrucks, vorzugsweise als Duftstoff.

8. Verwendung einer Verbindung der Formel (I) oder eines Stereoisomers davon oder eines Gemischs von Stereoisomeren davon oder eines Gemischs von verschiedenen Verbindungen der Formel (I) gemäß einem der vorhergehenden Ansprüche zur Modifizierung des Duftcharakters einer duftstoffhaltigen Zusammensetzung, insbesondere einer duftstoffhaltigen gebrauchsfertigen Zusammensetzung.

9. Verwendung nach einem der vorhergehenden Ansprüche in einer Zusammensetzung, die aus der Gruppe bestehend aus Parfümzusammensetzungen, Körperpflegezusammensetzungen, Produkten für die Mund- oder Zahnhygiene, Hygieneartikeln, Reinigungszusammensetzungen, Textilwaschmittelzusammensetzungen, Zusammensetzungen für Duftspender, Lebensmitteln, Nahrungsergänzungsmitteln, pharmazeutischen Zusammensetzungen und Pflanzenschutzzusammensetzungen ausgewählt ist.

10. Zusammensetzung, umfassend mindestens eine Verbindung der Formel (I), ein Stereoisomer davon oder ein Gemisch von Stereoisomeren davon gemäß einem der Ansprüche 1 bis 6 und mindestens eine weitere Komponente, die aus der Gruppe bestehend aus Aromachemikalien, die von den Verbindungen (I) verschieden sind, und Nicht-Aromachemikalien-Trägern ausgewählt ist;
wobei es sich bei den Trägermaterialien um flüssige oder ölartige oder feste Trägermaterialien handelt;
wobei die flüssigen oder ölartigen Trägermaterialien aus Methanol, Ethanol, Ethylenglykol, Glycerin, Diglycerin, Propylenglykol, Dipropylenglykol, 1,2-Butylenglykol, Hexamethylcyclotrisiloxan, Decamethylcyclopentasiloxan, Diethylenglykolmonoethylether, Diethylphthalat, Isopropylmyristat, Triethylcitrat, Benzylbenzoat, fraktioniertem Kokosnussöl, Tetradecylacetat, Tetradecyllactat, Estern des Glycerins mit Schmelztemperaturen unter 20 °C und Isopropylmyristat ausgewählt sind.

11. Zusammensetzung nach Anspruch 10, ausgewählt aus der Gruppe bestehend aus Parfümzusammensetzungen, Körperpflegezusammensetzungen, Produkten für die Mund- oder Zahnhygiene, Hygieneartikeln, Reinigungszusammensetzungen, Textilwaschmittelzusammensetzungen, Zusammensetzungen für Duftspender, Lebensmitteln, Nahrungsergänzungsmitteln, pharmazeutischen Zusammensetzungen und Pflanzenschutzzusammensetzungen.

12. Zusammensetzung, umfassend ein Gemisch von mindestens zwei Verbindungen, die aus der Gruppe bestehend aus Isomeren der Formeln (1.1), (I.2) und (1.3) ausgewählt sind, 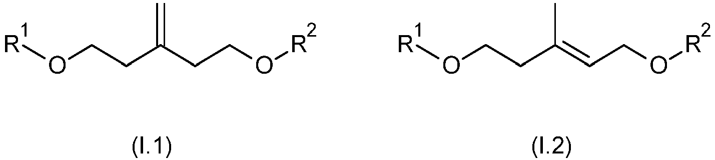 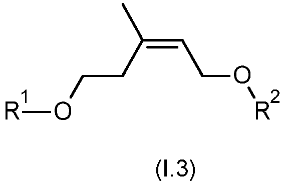
wobei in allen in dem Gemisch vorliegenden Verbindungen der Formeln (I.1), (I.2) und (I.3) alle Reste R¹ und R² gleich sind und entweder für C₁-C₄-Alkyl stehen oder alle Reste R¹ für -(C=O)-R³ stehen, alle Reste R² für -(C=O)-R⁴ stehen und alle Reste R³ und R⁴ gleich sind und für Wasserstoff oder C₁-C₄-Alkyl, vorzugsweise Wasserstoff, Methyl oder Ethyl, stehen;
umfassend jede der in dem Gemisch vorliegenden Verbindungen in einer Menge von 1 - 99 Gew.-%, mit der Maßgabe, dass sich das Gewicht der in dem Gemisch enthaltenen Verbindungen der Formeln (I.1), (1.2) und (1.3) zu 100 % addiert;
mit Ausnahme der folgenden Gemische:
- Gemisch von [(E)-5-Acetoxy-3-methylpent-3-enyl]acetat und [(Z)-5-Acetoxy-3-methylpent-3-enyl]acetat,
- Gemisch von [(E)-3-Methyl-5-propanoyloxypent-3-enyl]propanoat und [(Z)-3-Methyl-5-propanoyloxypent-3-enyl]propanoat,
- Gemisch von (E)-1,5-Dimethoxy-3-methylpent-2-en und (Z)-1,5-Dimethoxy-3-methylpent-2-en,
- Gemisch von (E)-1,5-Diethoxy-3-methylpent-2-en und (Z)-1,5-Diethoxy-3-methylpent-2-en,
- Gemisch von (E)-3-Methyl-1,5-dipropoxypent-2-en und (Z)-3-Methyl-1,5-dipropoxypent-2-en und
- Gemisch von (E)-1,5-Dibutoxy-3-methylpent-2-en und (Z)-1,5-Dibutoxy-3-methylpent-2-en.

13. Zusammensetzung nach Anspruch 12, enthaltend die Verbindung der Formel (I.1) und entweder eine der Verbindungen der Formeln (1.2) und (I.3) oder beide Verbindungen der Formeln (I.2) und (I.3) und insbesondere enthaltend alle drei Verbindungen (1.1), (1.2) und (1.3).

14. Zusammensetzung nach einem der Ansprüche 12 bis 13, umfassend ein Gemisch, das ausgewählt ist aus der Gruppe bestehend aus
- Gemischen von (5-Formyloxy-3-methylenpentyl)formiat, [(E)-5-Formyloxy-3-methylpent-3-enyl]formiat und [(Z)-5-Formyloxy-3-methylpent-3-enyl]formiat;
- Gemischen von (5-Acetoxy-3-methylenpentyl)acetat, [(E)-5-Acetoxy-3-methylpent-3-enyl]acetat und [(Z)-5-Acetoxy-3-methylpent-3-enyl]acetat;
- Gemischen von (3-Methylen-5-propanoyloxypentyl)propanoat, [(E)-3-Methyl-5-propanoyloxypent-3-enyl]propanoat und [(Z)-3-Methyl-5-propanoyloxypent-3-enyl]propanoat,
- Gemischen von 1,5-Dimethoxy-3-methylenpentan, (E)-1,5-Dimethoxy-3-methylpent-2-en und (Z)-1,5-Dimethoxy-3-methylpent-2-en,
- Gemischen von 1,5-Diethoxy-3-methylenpentan, (E)-1,5-Diethoxy-3-methylpent-2-en und (Z)-1,5-Diethoxy-3-methylpent-2-en und
- Gemischen von 1,5-Di-n-butoxy-3-methylenpentan, (E)-1,5-Di-n-butoxy-3-methylpent-2-en und (Z)-1,5-Di-n-butoxy-3-methylpent-2-en.

## Revendications

1. Utilisation d'un composé de formule (I) 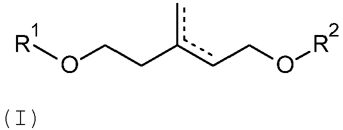 dans laquelle
l'un des symboles -̅ -̅ désigne une simple liaison et l'autre désigne une double liaison ou une simple liaison, et
R¹ et R² sont identiques et sont soit tous deux C₁-C₄-alkyle, soit R¹ est -(C=O)-R³ et R² est -(C=O)-R⁴, où R³ et R⁴ sont identiques et sont hydrogène ou C₁-C₄-alkyle, ou d'un stéréoisomère correspondant, ou d'un mélange de stéréoisomères correspondant, ou d'un mélange de différents composés de formule (I)
comme composé chimique aromatique.

2. Utilisation selon la revendication 1, où dans la formule (I)
R¹ est C₁-C₄-alkyle,
R² est C₁-C₄-alkyle.

3. Utilisation selon la revendication 1 ou 2, où le composé de formule (I) possède au moins l'une des caractéristiques suivantes b) ou c)
b) R¹ et R² sont C₁-C₃-alkyle,
c) R¹ et R² sont méthyle ou éthyle, préférablement méthyle.

4. Utilisation selon la revendication 1, où dans la formule (I)
R¹ est -(C=O)-R³, et
R² est -(C=O)-R⁴

5. Utilisation selon la revendication 4, où R³ et R⁴ sont hydrogène ou C₁-C₃-alkyle, préférablement hydrogène, méthyle ou éthyle.

6. Utilisation selon l'une quelconque des revendications précédentes, d'un mélange contenant deux ou trois isomères d'un composé de formule I, les isomères étant des formules (1.1), (1.2) et (1.3),

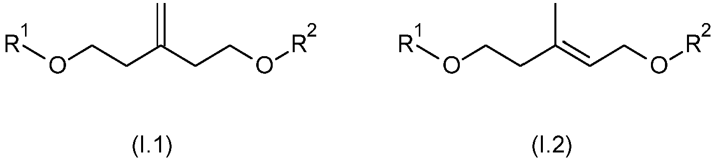

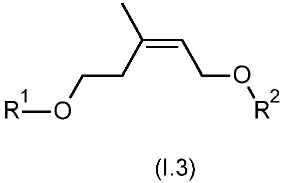

7. Utilisation selon l'une quelconque des revendications précédentes, pour conférer une impression olfactive, de préférence comme fragrance.

8. Utilisation d'un composé de formule (I) ou d'un stéréoisomère correspondant ou d'un mélange de stéréoisomères correspondant ou d'un mélange de différents composés de formule (I) tels que définis dans l'une quelconque des revendications précédentes, pour modifier le caractère parfumé d'une composition fragrancée, en particulier d'une composition fragrancée prête à l'emploi.

9. Utilisation selon l'une quelconque des revendications précédentes, dans une composition choisie dans le groupe constitué par les compositions de parfum, les compositions de soins corporels, les produits d'hygiène bucco-dentaire, les articles d'hygiène, les compositions de nettoyage, les compositions de détergent pour textiles, les compositions pour diffuseurs de parfum, les produits alimentaires, les compléments alimentaires, les compositions pharmaceutiques et les compositions de protection de cultures.

10. Composition comprenant au moins un composé de formule (I), un stéréoisomère correspondant ou un mélange de stéréoisomères correspondant, tel que défini dans l'une quelconque des revendications 1 à 6, et au moins un autre composant choisi dans le groupe constitué par des composés chimiques aromatiques différents des composés (I) et des supports chimiques non aromatiques ; où les matières de support sont liquides ou de type huile, ou des matières de support solides ;
où les matières de support liquides ou de type huile sont choisies parmi le méthanol, l'éthanol, l'éthylène glycol, le glycérol, le diglycérol, le propylène glycol, le dipropylène glycol, le 1,2-butylène glycol, l'hexaméthylcyclotrisiloxane, le décaméthylcyclopentasiloxane, l'éther monoéthylique de diéthylène glycol, le phtalate de diéthyle, le myristate d'isopropyle, le citrate de triéthyle, le benzoate de benzyle, l'huile de coco fractionnée, l'acétate de tétradécyle, le lactate de tétradécyle, les esters de glycérol ayant des températures de fusion inférieures à 20 °C et le myristate d'isopropyle.

11. Composition selon la revendication 10, choisie dans le groupe constitué par les compositions de parfum, les compositions de soins corporels, les produits d'hygiène bucco-dentaire, les articles d'hygiène, les compositions de nettoyage, les compositions de détergent pour textiles, les compositions pour diffuseurs de parfum, les produits alimentaires, les compléments alimentaires, les compositions pharmaceutiques et les compositions de protection de cultures.

12. Composition comprenant un mélange d'au moins deux composés choisis dans le groupe constitué par les isomères de formules (1.1), (1.2) et (1.3), 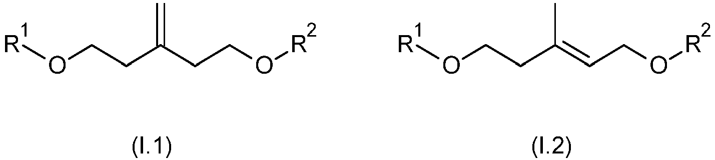 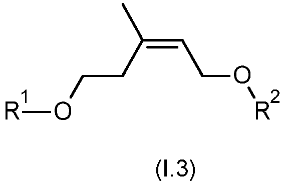
où, dans tous les composés de formules (1.1), (1.2) et (1.3) présents dans le mélange, tous les radicaux R¹ et R² sont identiques et sont soit C₁-C₄-alkyle, soit tous les radicaux R¹ sont -(C=O)-R³, tous les radicaux R² sont -(C=O)-R⁴, et tous les radicaux R³ et R⁴ sont identiques et sont hydrogène ou C₁-C₄-alkyle, de préférence hydrogène, méthyle ou éthyle ;
comprenant chacun des composés présents dans le mélange en une quantité de 1 à 99 % en poids, à condition que le poids des composés de formules (1.1), (1.2) et (1.3) présents dans ledit mélange totalise 100 % ;
sauf pour les mélanges suivants :
- mélange d'acétate de [(E)-5-acétoxy-3-méthyl-pent-3-ényle] et d'acétate de [(Z)-5-acétoxy-3-méthyl-pent-3-ényle],
- mélange de propanoate de [(E)-3-méthyl-5-propanoyloxy-pent-3-ényle] et de propanoate de [(Z)-3-méthyl-5-propanoyloxy-pent-3-ényle],
- mélange de (E)-1,5-diméthoxy-3-méthyl-pent-2-ène et de (Z)-1,5-diméthoxy-3-méthyl-pent-2-ène,
- mélange de (E)-1,5-diéthoxy-3-méthyl-pent-2-ène et de (Z)-1,5-diéthoxy-3-méthyl-pent-2-ène,
- mélange de (E)-3-méthyl-1,5-dipropoxy-pent-2-ène et de (Z)-3-méthyl-1,5-dipropoxy-pent-2-ène, et
- mélange de (E)-1,5-dibutoxy-3-méthyl-pent-2-ène et de (Z)-1,5-dibutoxy-3-méthyl-pent-2-ène.

13. Composition selon la revendication 12, contenant le composé de formule (1.1) et l'un ou l'autre des composés de formules (1.2) et (1.3) ou les deux composés de formules (1.2) et (1.3) ; et contenant en particulier les trois composés (1.1), (1.2) et (I.3).

14. Composition selon l'une quelconque des revendications 12 à 13, comprenant un mélange qui est choisi dans le groupe constitué par
- mélanges de formiate de (5-formyloxy-3-méthylène-pentyle), de formiate de [(E)-5-formyloxy-3-méthyl-pent-3-ényle] et de formiate de [(Z)-5-formyloxy-3-méthyl-pent-3-ényle] ;
- mélanges d'acétate de (5-acétoxy-3-méthylène-pentyle), d'acétate de [(E)-5-acétoxy-3-méthyl-pent-3-ényle] et d'acétate de [(Z)-5-acétoxy-3-méthyl-pent-3-ényle] ;
- mélanges de propanoate de (3-méthylène-5-propanoyloxy-pentyle), de propanoate de [(E)-3-méthyl-5-propanoyloxy-pent-3-ényle] et de propanoate de [(Z)-3-méthyl-5-propanoyloxy-pent-3-ényle],
- mélanges de 1,5-diméthoxy-3-méthylène-pentane, de (E)-1,5-diméthoxy-3-méthyl-pent-2-ène et de (Z)-1,5-diméthoxy-3-méthyl-pent-2-ène,
- mélanges de 1,5-diéthoxy-3-méthylène-pentane, de (E)-1,5-diéthoxy-3-méthyl-pent-2-ène et de (Z)-1,5-diéthoxy-3-méthyl-pent-2-ène, et
- mélanges de 1,5-di-n-butoxy-3-méthylène-pentane, de (E)-1,5-di-n-butoxy-3-méthyl-pent-2-ène et de (Z)-1,5-di-n-butoxy-3-méthyl-pent-2-ène.
